# EUROPEAN PATENT SPECIFICATION

(11) **EP 0 854 899 B1**
(45) Date of publication and mention of the grant of the patent: **10.01.2001**
(21) Application number: 96932809.5
(22) Date of filing: 02.10.1996
(51) Int. Cl.: C09K 19/04, C09K 19/30, C09K 19/12, C09K 19/34, C09K 19/40, C09K 19/42, C07C 22/00, C07C 22/08, C07C 25/24, C07C 43/225, C07C 43/192

(54) **FLUORINE-SUBSTITUTED ALKENYL LIQUID CRYSTALLINE COMPOUNDS**
FLUORSUBSTITUIERTE ALKENYLFLÜSSIGKRISTALLVERBINDUNGEN
COMPOSES CRISTALLINS LIQUIDES ALCENYLE A SUBSTITUTION FLUOR

(30) Priority: 09.10.1995 JP 28791795
(43) Date of publication of application: 29.07.1998
(73) Proprietor: CHISSO CORPORATION, Osaka-shi, Osaka-fu 530 (JP)
(72) Inventor: OHNISHI, Noriyuki, Tsukuba-shi, Ibaraki (JP); MATSUI, Shuichi, Chiba 290 (JP); MIYAZAWA, Kazutoshi, Chiba 290-01 (JP); SEKIGUCHI, Yasuko, Chiba 290 (JP); NAKAGAWA, Etsuo, Chiba 290 (JP)
(74) Representative: Hansen, Bernd, Dr. Dipl.-Chem.
(86) International application number: JP9602878
(87) International publication number: WO9713821

(56) References cited:
- EP-A- 0 104 327
- EP-A- 0 167 912
- EP-A- 0 315 014
- EP-A- 0 390 395
- EP-A- 0 563 982
- EP-A- 0 688 850
- DE-A- 4 211 694
- DE-A- 4 223 058
- DE-A- 4 238 377

## Description

The present invention relates to novel liquid crystalline compounds showing preferable physical properties and liquid crystalline compositions having preferable physical properties obtained by using the above-mentioned novel liquid crystalline compounds.

Liquid crystalline display elements utilize optical anistropy and dielectric anistropy, both of which are possessed by liquid crystalline compounds, and there are known a twist nematic type (TN type), a supertwist nematic type (STN type), a dynamic scattering type (DS type) and a guest/host type (G/H type) etc. as display methods therefor. Also, there are known a static driving method, a time-separating driving method, an active matrix driving method and a 2-frequency driving method etc. as driving methods threrfor.

Properties of liquid crystalline compounds used in these various liquid crystalline display elements are different according to their applications, but any of the liquid crystalline compounds is required to be stable against external environmental factors such as water content, air, heat and light etc., and also to show a liquid crystalline phase within a high range as possible, preferably with centering around the room temperature.

Furthermore, in order that the liquid crystalline compounds show optimal properties required for individual display elements, the liquid crystalline compositions are consistuted from several or more than 20 liquid crystalline compounds. Thus, they are required to be compatible with other liquid crystalline compounds, and in particular recently required to have good compatibility at low temperatures because of requests resulted from uses under various environments.

Compositions used in a STN driving method which are most practiced recently are particularly required to have sharp threshold value characteristics in order to realize high image qualities. It is known that the said sharpness is a function of an elastic constant ratio K33/K11, wherein the more the liquid crystalline compound has the constant ratio, the sharper the liquid crystalline compsition which uses the said compound has the threshold value (F. Leenhouts and et al., Proceedings of the Japan Display 388 (1986)).

Furthermore, it is necessary to use compositions with rapid response speed in order to realize enhighment of display element planes. The response speed is known to be a function of viscosity (E. Jakeman and et al., Phys. Lett., 39A, 69 (1972)). That is, it is important to use liquid crystalline compounds with low viscosity, which is required to realize compositions with low viscosity.

The following compounds having alkenyl sites are known as compounds with high elastic constant ratio K33/K11. That is, there may be mentioned 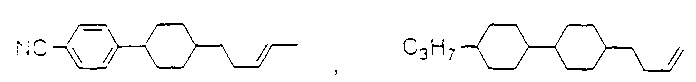 described in Mol. Cryst. Liq. Cryst., 122 (1985) and Toku-Kai-Sho 61-83136, or 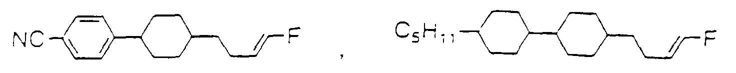 having an introduced fluorine atom described in Toku-Gan-Hei 6-92740.

Any of these alkenyl compounds has high elastic constant ratio K33/K11, and any of liquid crystalline compositions obtained by using them has comparatively preferable sharpness. However, liquid crystalline compositions showing more rapid response speed, in other words, the compositions showing lower viscosity, are required owing to increase in requests for display ability of display elements.

Furthermore, compounds 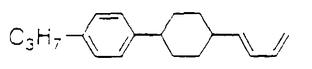 having 1,3-butadienyl groups described in Toku-Gan-Hei 6-151445 are known as compounds with high elastic constant ratio K33/K11.

However, the substituent, a 1,3-butadienyl group, is very unstable chemically, so that it is impossible to use them for practical liquid crystalline compositions.

Furthermore, dienyl compounds having multiple double bonds in one same chain are disclosed in Toku-Gan-Hei 7-26029. Although the compound with high elastic constant ratio K33/K11 and high stability are disclosed in the said invention, it is impossible to prepare liquid crystalline compositions having low viscosity and sufficiently rapid response property.

DE 42 23 058-A1 reveals mesogenic polyenes comprising at one terminal group conjugated carbon-carbon-double bonds. These compounds are used in liquid crystalline compositions and have a high cleaning point and a low viscosity.

EP-0 563 982-A2 discloses polycyclic diene derivatives comprising a carbon chain containing a conjugated diene as a terminal substituent. These compounds have a generally usable viscosity and a high NI point and a high elastic constant ratio. A liquid crystal composition comprising such a compound is particularly suitable for STN mode.

Thus, liquid crystalline compounds which have high elastic constant ratio K33/K11, lower viscosity than the conventional liquid crystalline compounds, high chemical stability, and good compatibility with other liquid crystalline compounds are expected.

An object of the invention is to propose novel liquid crystalline compounds having high elastic constant ratio, lower viscosity compared to known liquid crystalline compounds, superior compatibility with other liquid crystalline compounds, particularly compatibility at low temperatures, and chemical stability as well as liquid crystalline compositions containing the said composition.

The present invention describes a liquid crystalline compound, a liquid crystalline composition containing the same and a liquid crystalline display element as defined in the patent claims.

Preferable compounds amongst the compounds (1) are compound groups expressed by the general formula groups (1-a) ∼ (1-f). In the following formulae, W and W' are substituents given in the general formula (1-1) , and R₁, X₁, X₂, X₃, as well as rings A, B, C and D have the same meanings as the above-mentioned ones.

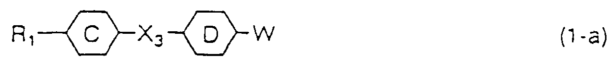

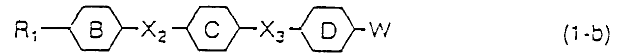

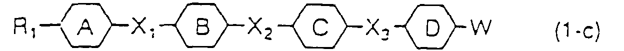

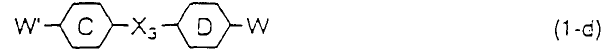

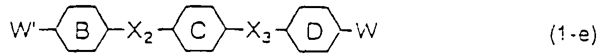

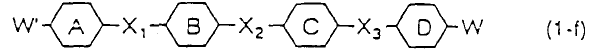

Furthermore, particularly preferable compounds amongst the general formula groups (1-a) ∼ (1-f) are the following compounds. 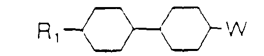 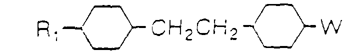 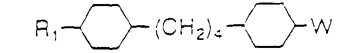 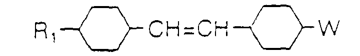 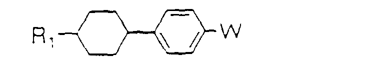 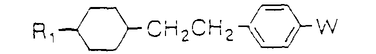 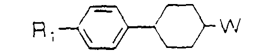 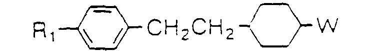 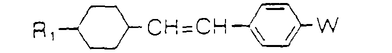 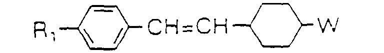 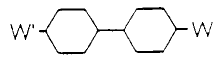 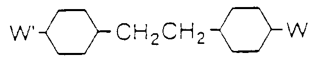 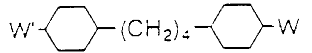 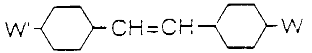 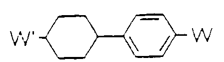 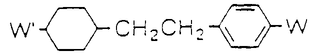 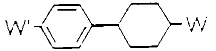 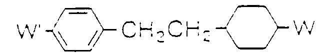 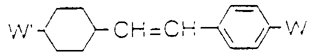 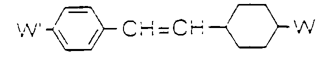 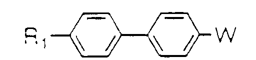 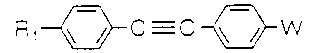 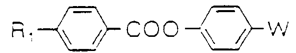 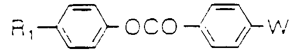 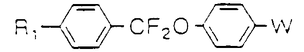 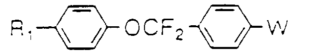 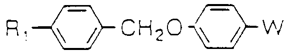 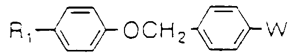 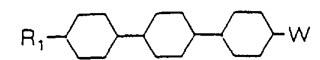 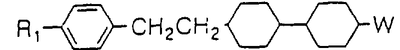 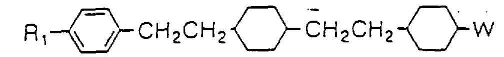 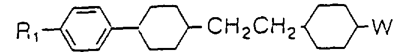 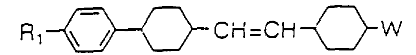 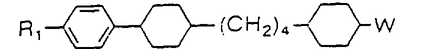 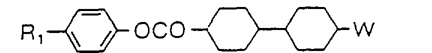 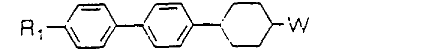 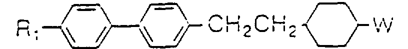 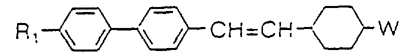 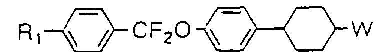 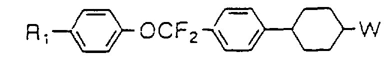 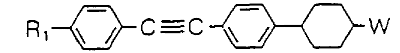 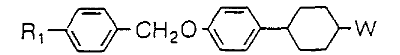 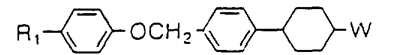 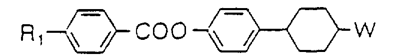 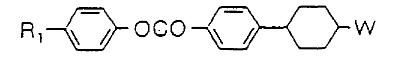 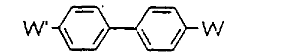 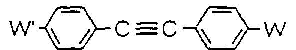 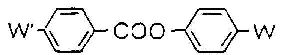 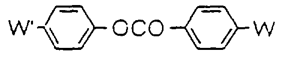 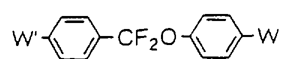 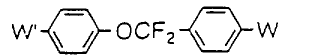 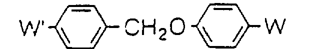 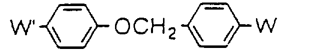 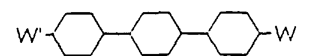 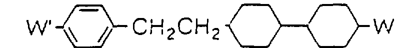 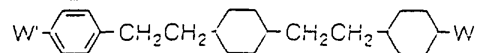 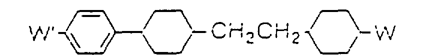 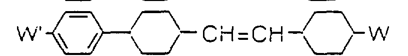 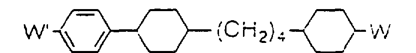 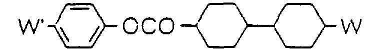 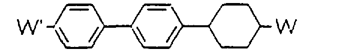 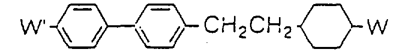 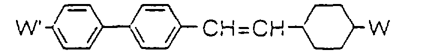 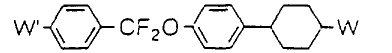 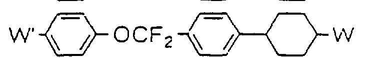 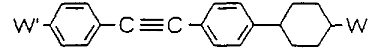 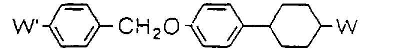 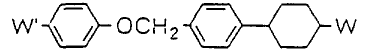 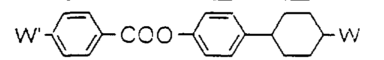 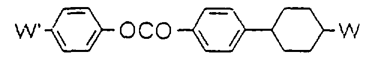 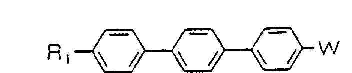 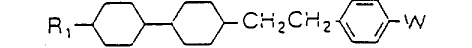 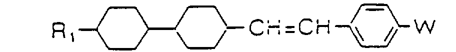 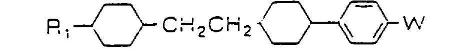 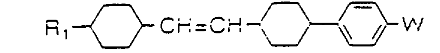 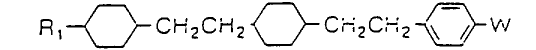 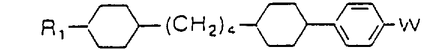 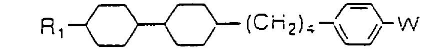 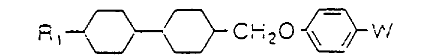 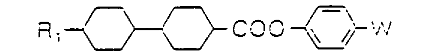 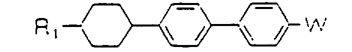 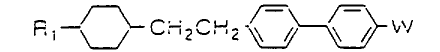 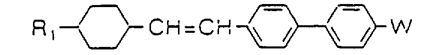 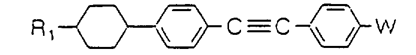 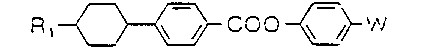 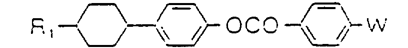 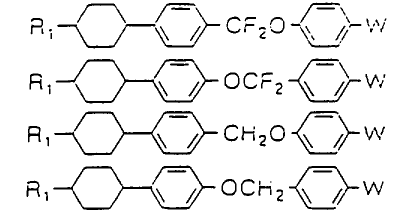 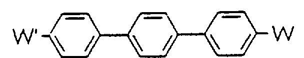 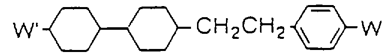 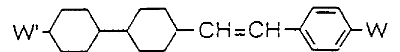 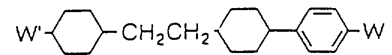 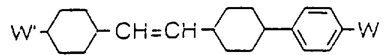 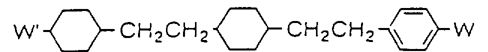 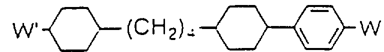 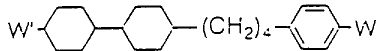 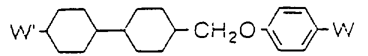 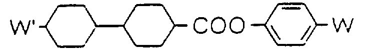 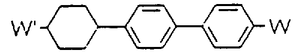 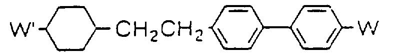 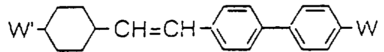 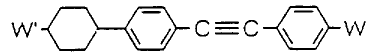 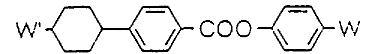 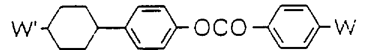 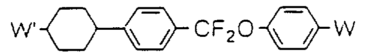 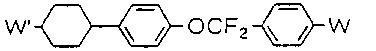 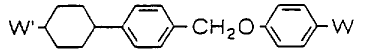 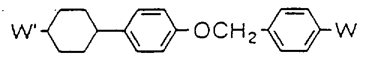 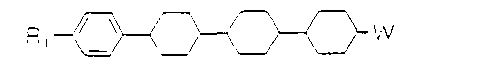 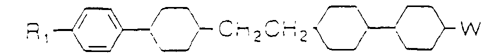 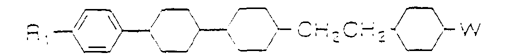 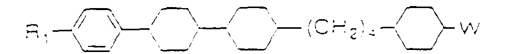 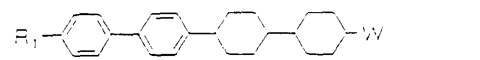 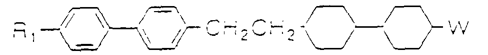 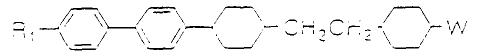 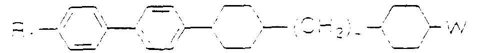 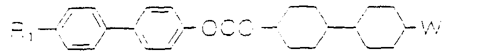 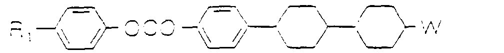 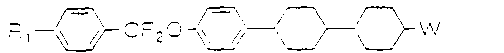 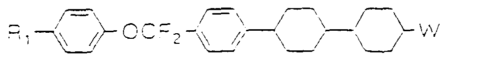 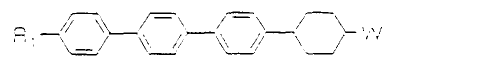 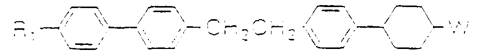 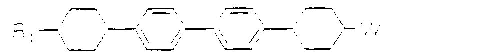 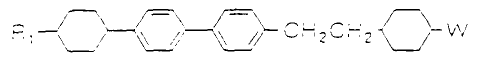 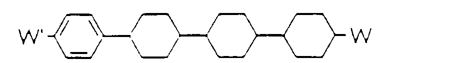 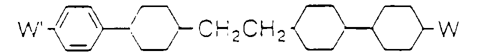 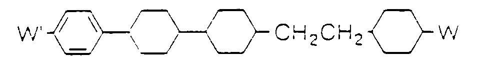 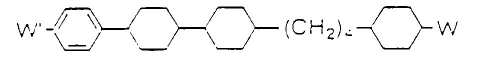 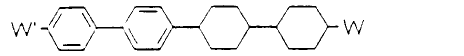 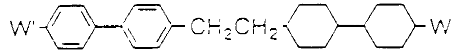 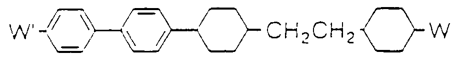 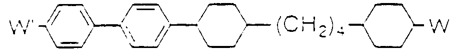 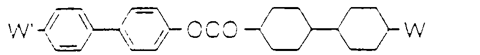 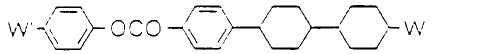 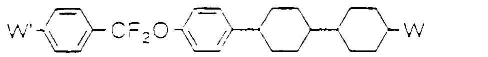 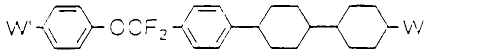 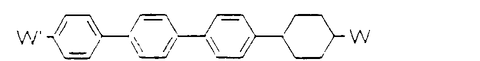 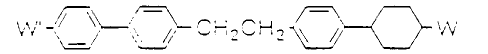 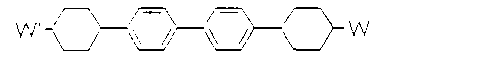 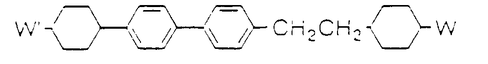 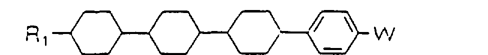 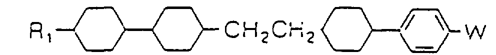 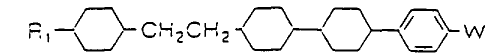 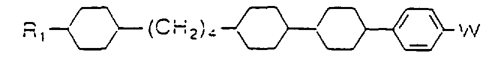 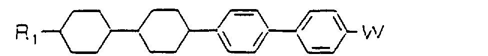 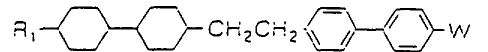 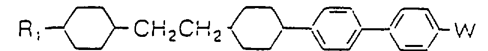 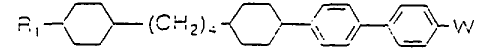 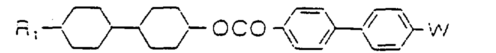 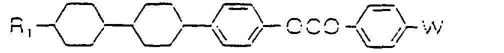 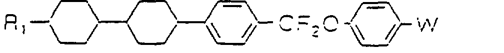 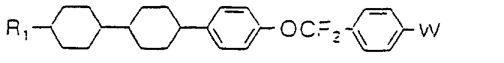 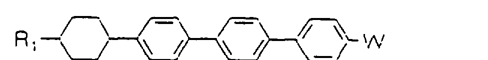 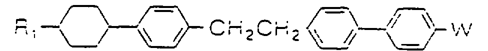 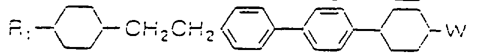 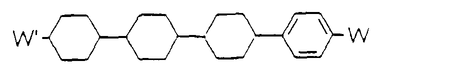 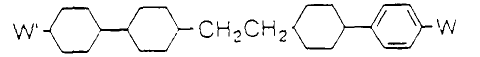 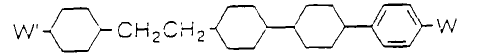 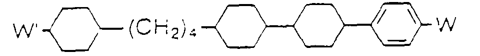 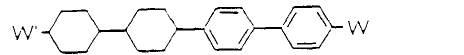 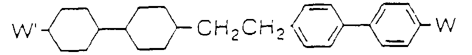 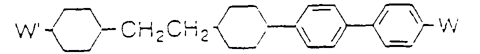 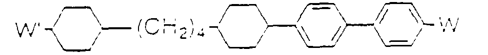 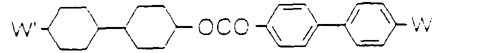 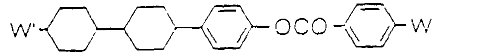 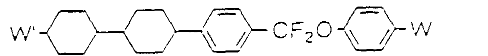 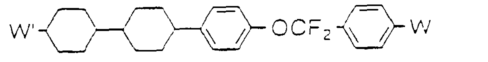 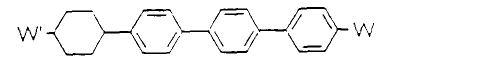 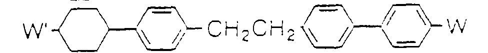 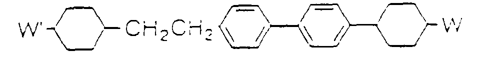 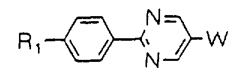 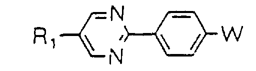 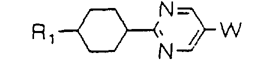 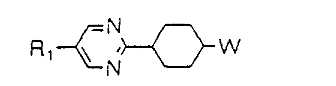 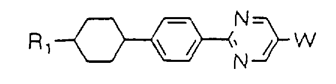 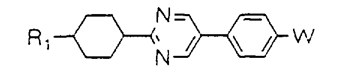 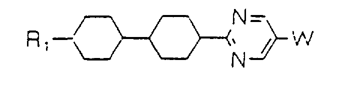 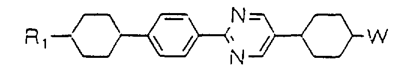 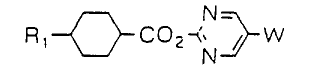 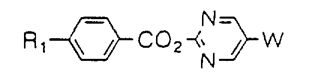 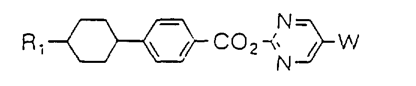 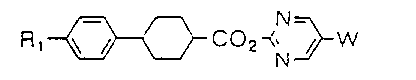 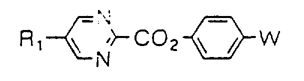 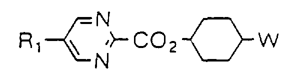 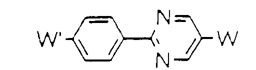 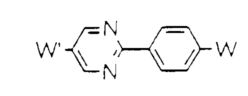 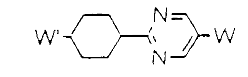 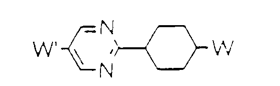 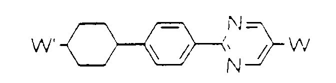 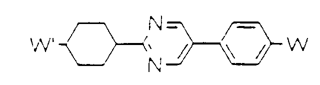 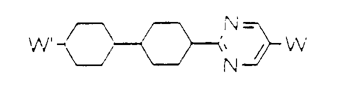 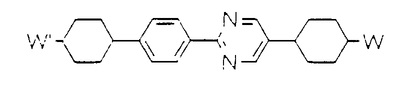 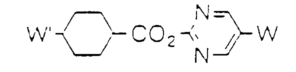 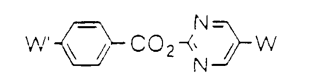 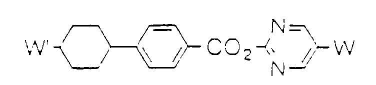 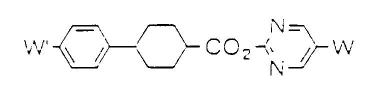 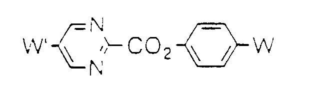 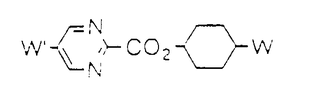 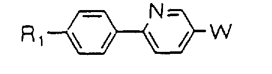 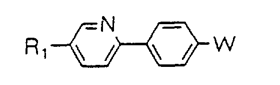 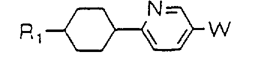 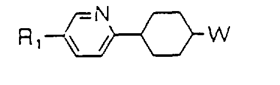 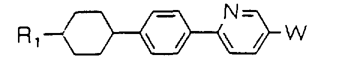 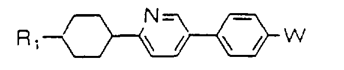 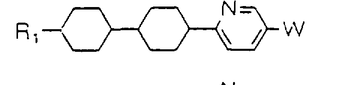 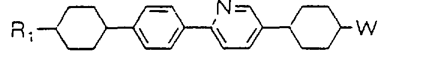 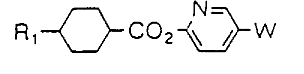 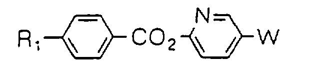 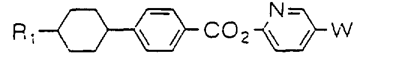 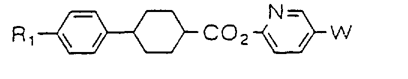 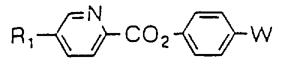 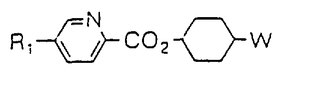 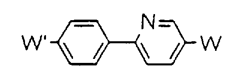 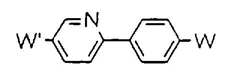 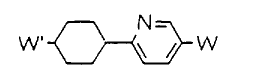 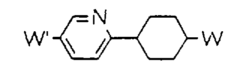 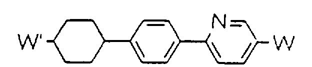 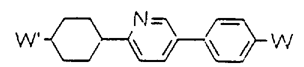 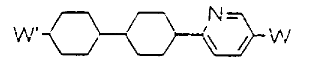 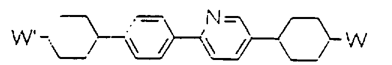 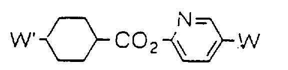 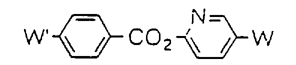 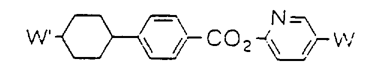 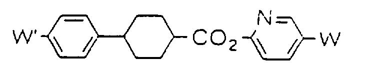 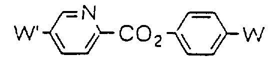 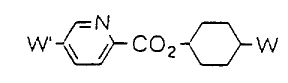 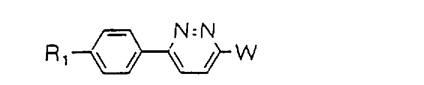 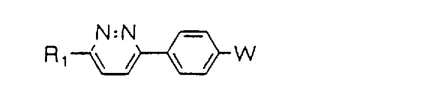 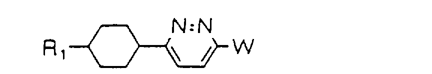 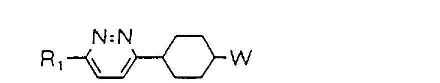 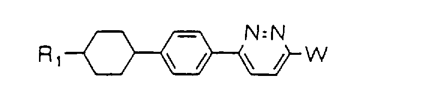 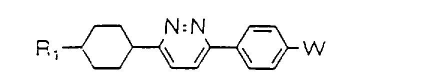 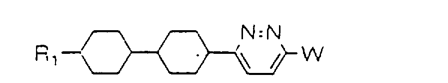 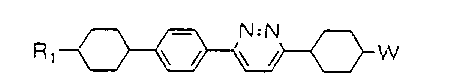 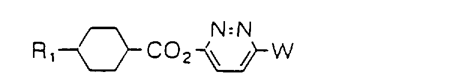 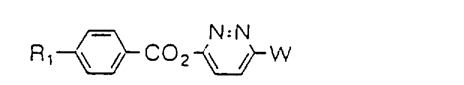 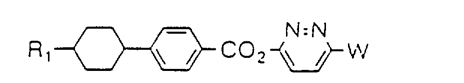 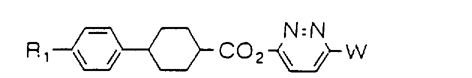 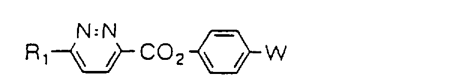 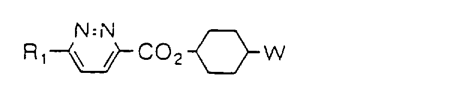 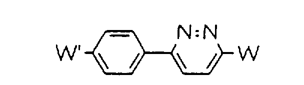 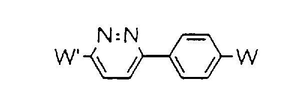 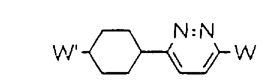 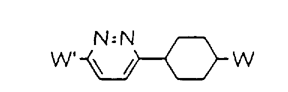 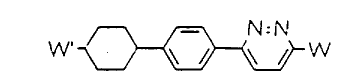 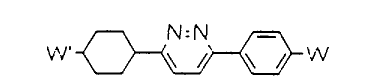 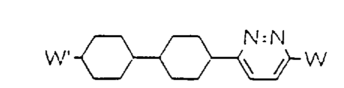 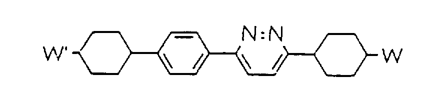 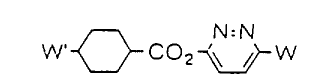 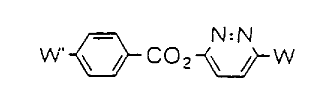 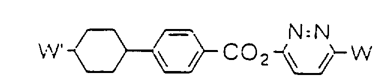 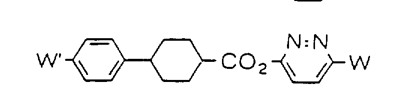 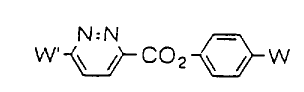 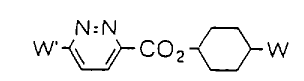 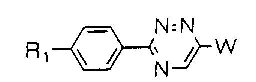 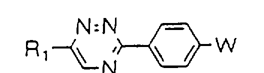 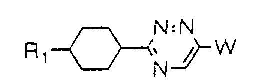 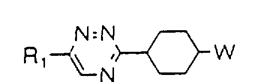 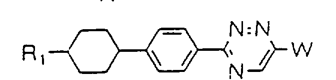 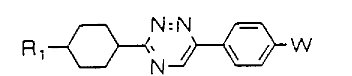 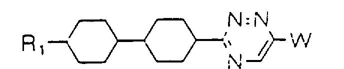 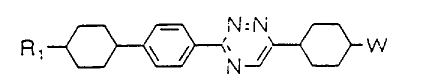 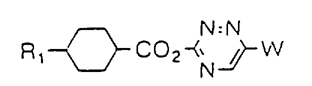 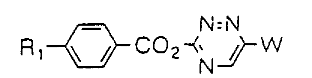 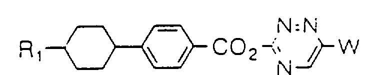 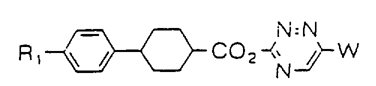 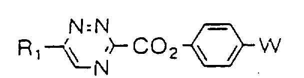 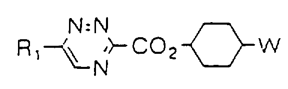 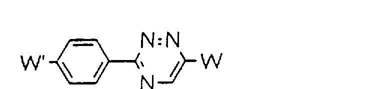 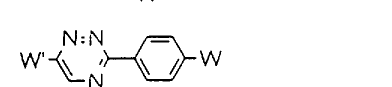 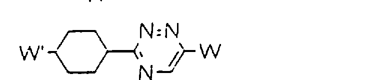 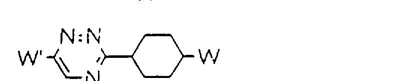 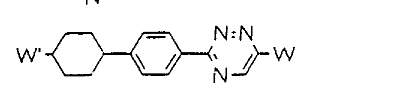 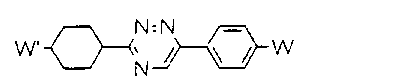 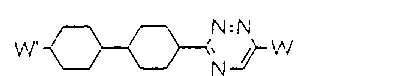 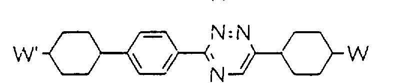 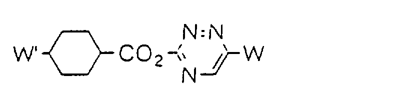 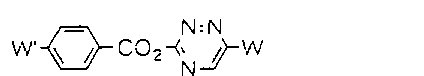 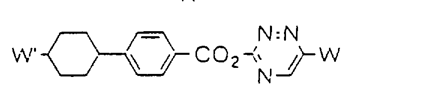 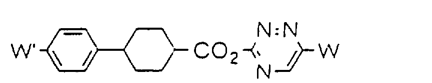 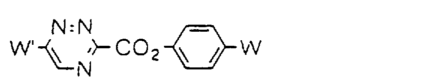 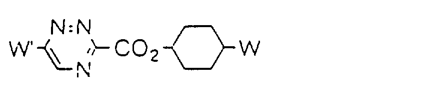 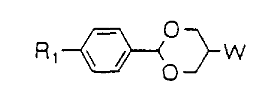 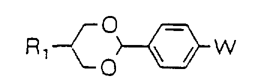 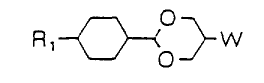 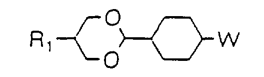 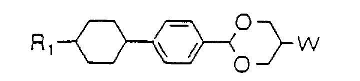 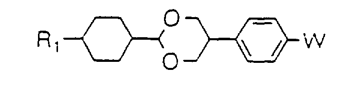 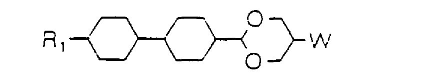 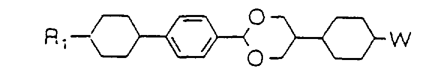 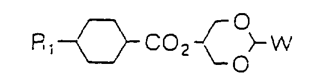 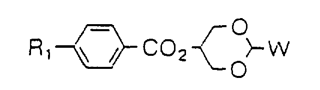 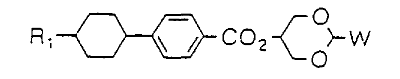 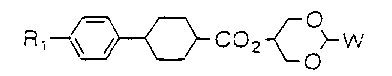 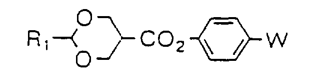 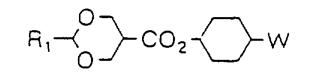 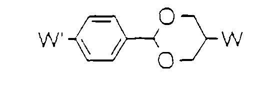 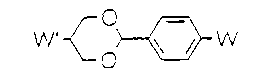 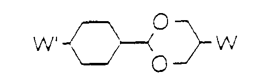 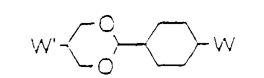 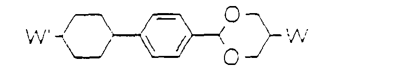 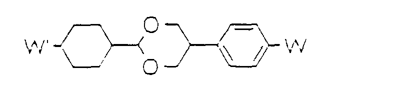 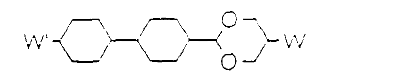 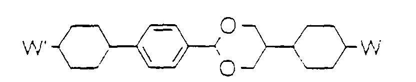 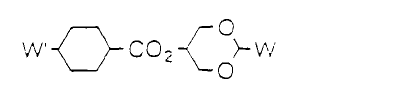 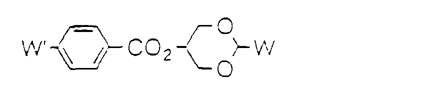 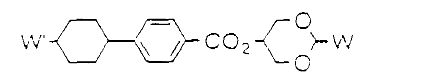 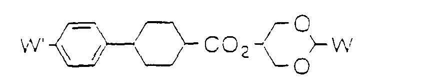 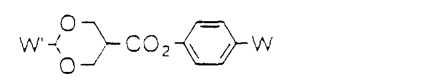 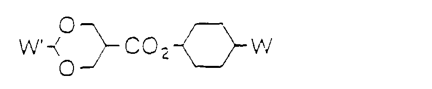 (In the above formulae, R₁ and W denote the same meanings as the above-mentioned ones, wherein a 1,4-phenylene ring, a trans-1,4-cyclohexylene ring, a bicycio[1.1.0]butane ring, a bicyclo-[1.1.1]pentane ring, a bicyclo[2.2.2]octane ring, a cyclobutane ring and a spiro[3,3]heptane ring may be substituted with (a) halogen atom(s), and (a) carbon atom(s) in the said rings may be substituted with (a) nitrogen atom(s) or (an) oxygen atom(s).)

Any of W and W shows preferable characteristics if satisfying the conditions shown in the general formula (1-1), but the following W1 ∼ W24 are particularly preferable.

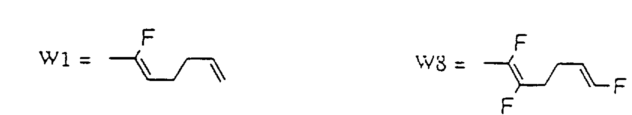

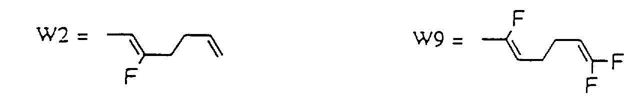

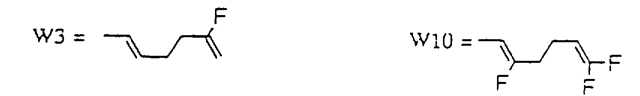

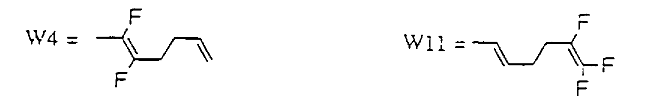

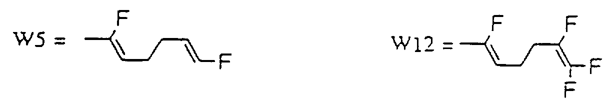

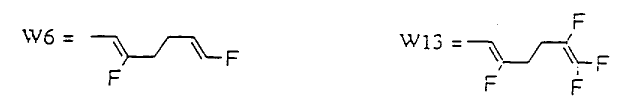

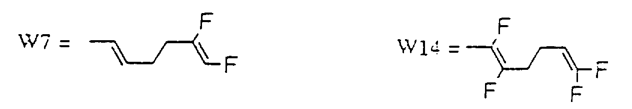

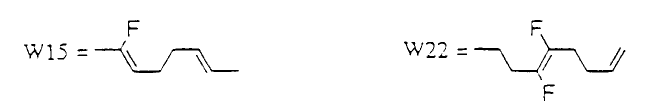

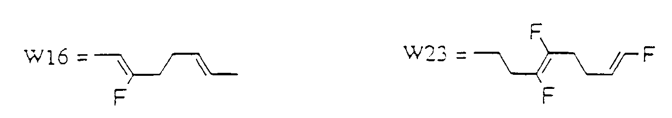

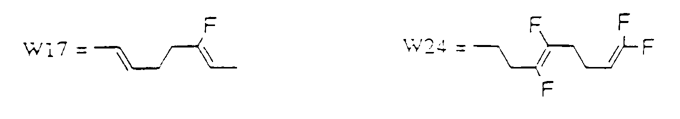

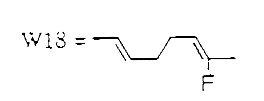

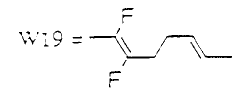

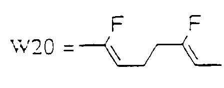

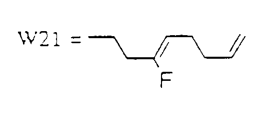

In the case of considering stability in particular, W and W' in which q is 2 or more than 2 are preferable.

Furthermore, R₁ and R₂ show the above-mentioned W, W', a cyano group, a halogen atom, an alkyl group with from 1 to 20 carbon atoms or a halogenated alkyl group with from 1 to 20 carbon atoms, wherein (a) methylene group(s) in the said groups may be substituted with (an) oxygen atom(s) or -C=C-(s). A fluorine atom and a chlorine atom are preferable as halogen atoms, and alkyl groups with from 1 to 10, particularly from 1 to 6, carbon atoms are preferable as alkyl groups.

Furthermore, as halogenated alkyl groups, trifluoromethyl, trifluoromethoxy, difluoromethoxy, 2-fluoroethyl, 2,2-difluoroethyl, 2,2,2-trifluoroethyl, 3-fluoropropyl, 2-fluoropropyl, 2,2-difluoropropyl, 4-fluorobutyl, 3-fluorobutyl, 2-fluorobutyl, 3,3-difluorobutyl, 2,2-difluorobutyl, 5-fluoropentyl, 4-fluoropentyl, 3-fluoropentyl, 3,3-difluoropentyl and 10-fluorodecyl are preferable. As those in which a methylene group being substituted with an oxygen atom, methoxymethyl, ethoxymethyl, propoxymethyl, butoxymethyl, pentoxymethyl, methoxyethoxy, methoxypropyl, ethoxyethoxy and ethoxypropoxyl groups are preferable. As those in which a methylene group being substituted with -CH=CH-. vinyl, 1-propenyl, 2-propenyl, 1-butenyl, 3-butenyl, 1-pentenyl, 2-pentenyl and 3-pentenyl are preferable.

Any compound of the formula (1) according to the invention has high elastic constant ratio K33/K11 and low viscosity. Furthermore, any compound is quite stable chemically.

Although any of the compounds according to the invention shows preferable physical properties, liquid crystalline compositions having properties according to their objects can be prepared by using compounds in which R₁, R₂, X₁, X₂, X₃, X₄, X₅, X₆, X₇, X₈, A, B, C, D, m, n, p and q are appropriately selected. That is, in the case of using the compounds for compositions in which the liquid crystalline temperature range being particularly within higher temperature sides, 3-ring type or 4-ring type compounds can be used, and if not, 2-rings type or 3-ring type compounds can be used.

In the case to be required particularly high dielectric anisotropy values, compounds with positive dielectric anisotropy value (P type compounds) are used, and P type compounds may be obtained by selecting a halogen atom, a cyano group or a halogenated alkyl group as R₁ in the formula (1). In the case to be required higher dielectric anisotropy value, the object may be attained by introducing a halogen atom on a ring bonded to R₁. In order to obtain compounds with negative dielectric anisotropy value (N type compounds), groups without high dipole moment such as an alkyl group may be introduced.

Optical index anisotropy values can be also controlled by selecting R₁, R₂, X₁, X₂, X₃, X₄, X₅, X₆, X₇, X₈, A, B, C, D, m, n, p and q in the general formula (1) optionally. That is, in the case to be required high refractive index anisotropy values, compounds containing much 1,4-phenylene rings may be used, and in the case to be required low optical index anisotropy values, compounds containing much 1,4-cyclohexylene rings may be used.

A liquid crystalline composition according to the invention preferably contains one or more than one compound(s) shown by (1) at a proportion of 0.1 ∼ 99% by weight, in order to realize superior properties.

In more detail, a liquid crystalline composition proposed by the invention is accomplished by adding the first component containing at least one compound (1) and if required then mixing a compound optionally selected from the compound groups expressed by the general formulae (2) ∼ (9) according to the object of the liquid crystalline composition.

As compounds expressed by the general formulae (2) ∼ (4) used in the invention, the following compounds may be preferably mentioned. (Wherein, R₃ denotes the same meaning as the above one.)

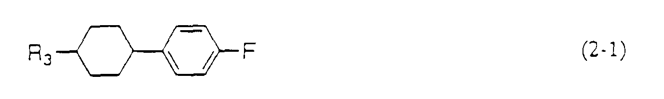

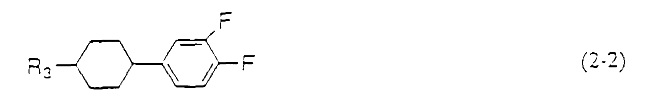

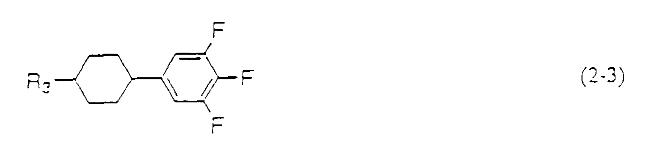

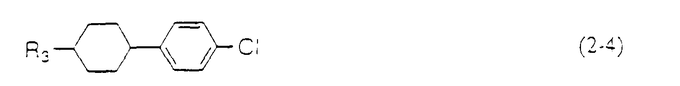

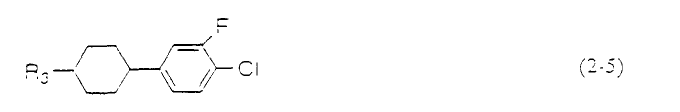

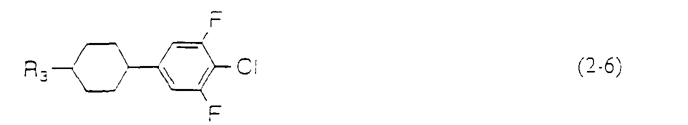

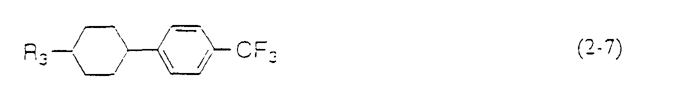

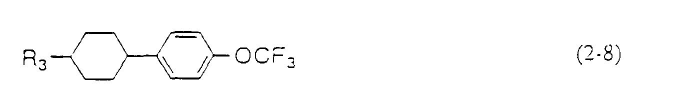

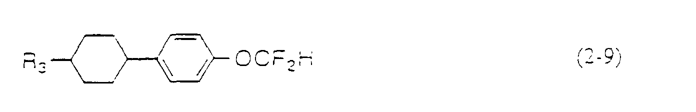

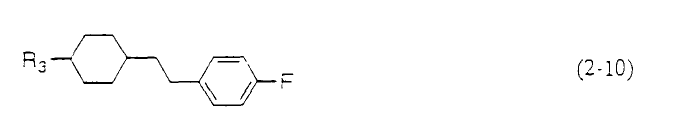

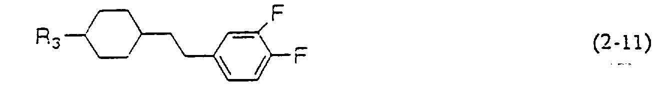

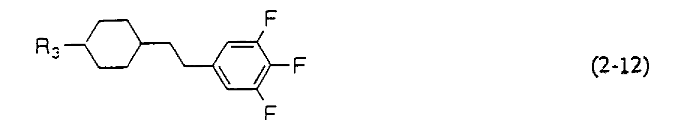

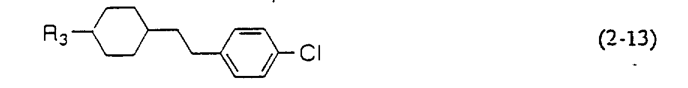

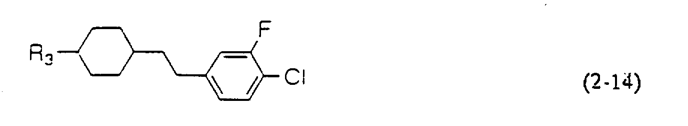

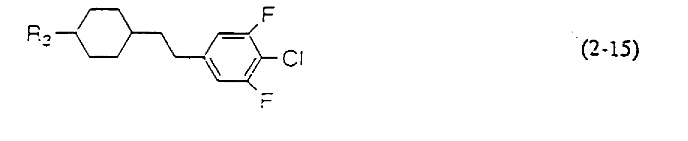

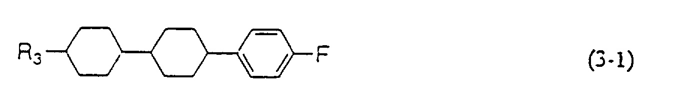

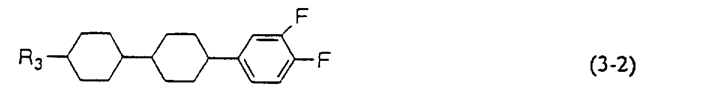

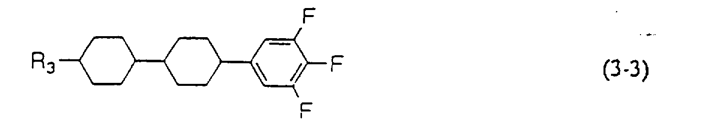

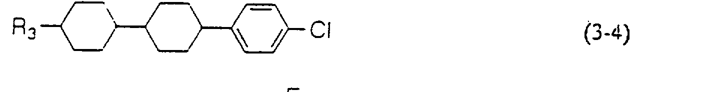

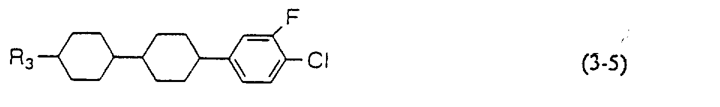

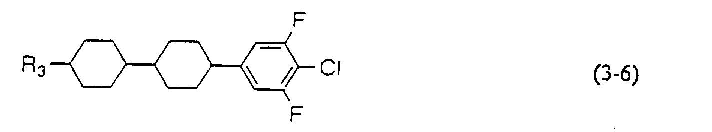

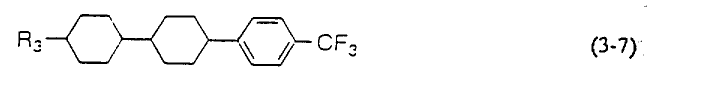

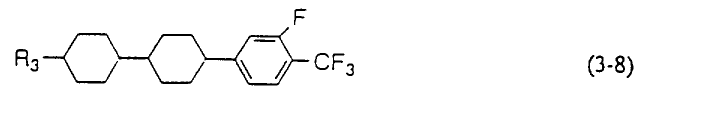

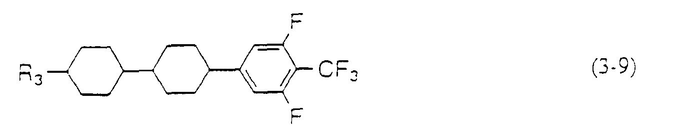

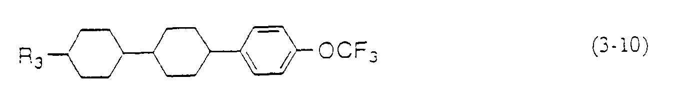

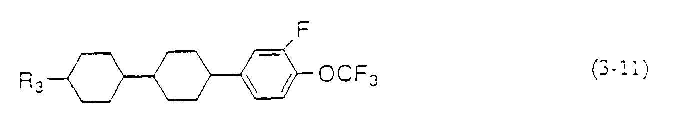

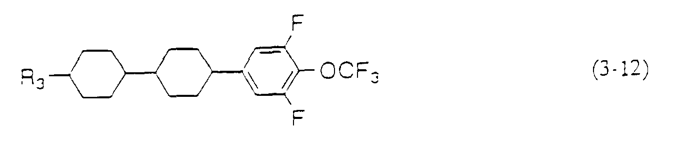

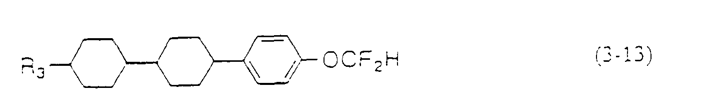

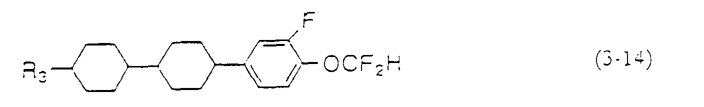

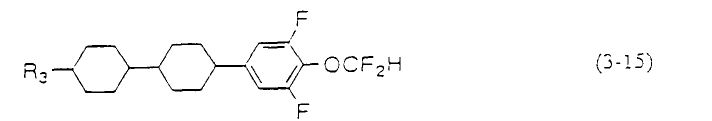

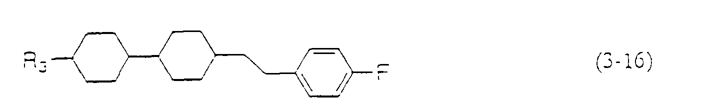

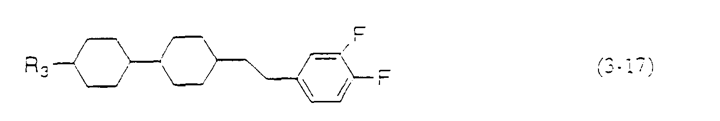

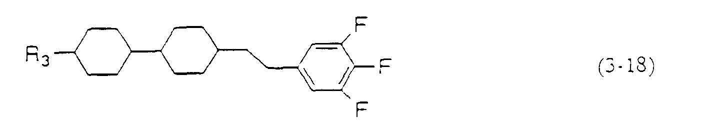

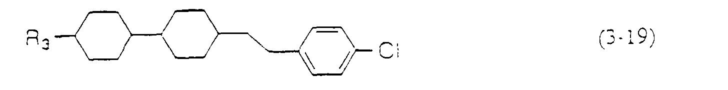

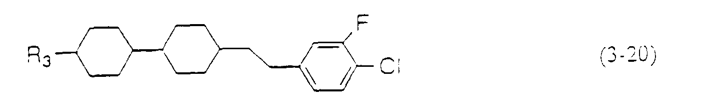

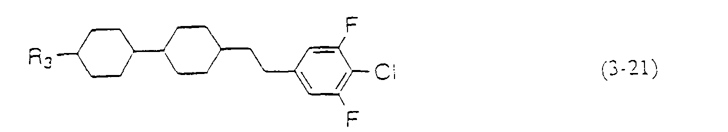

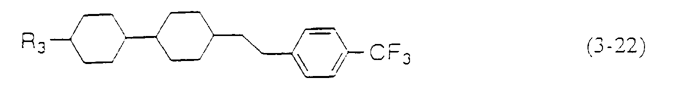

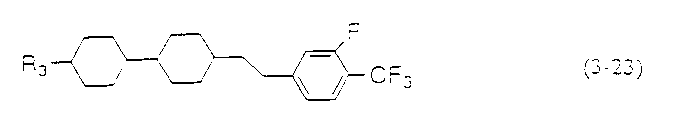

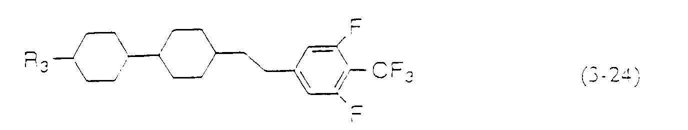

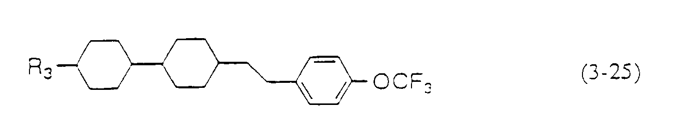

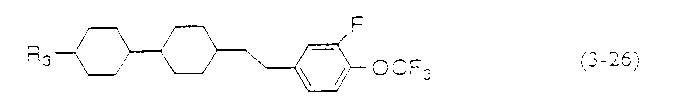

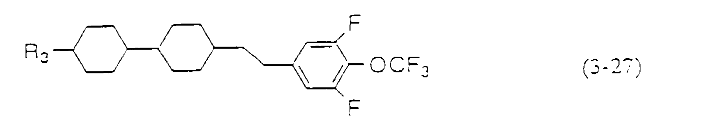

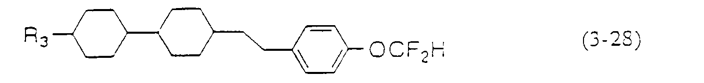

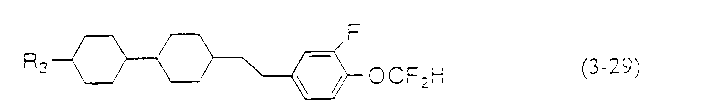

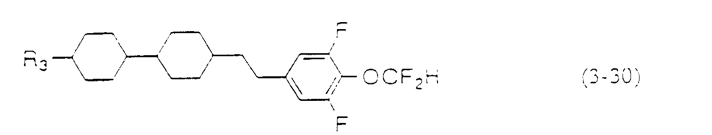

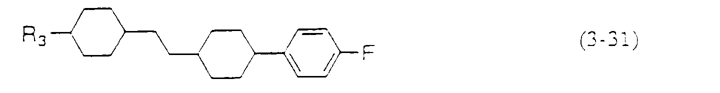

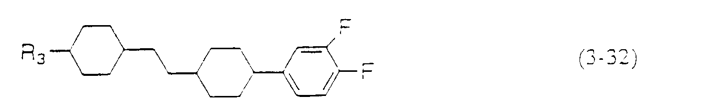

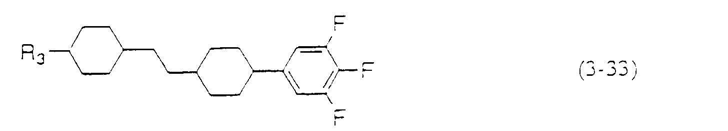

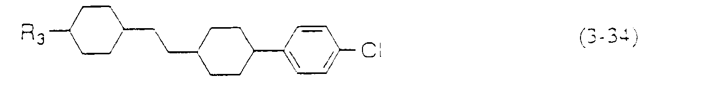

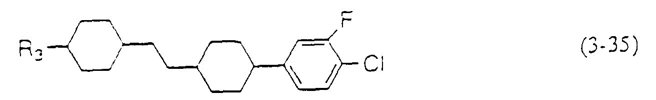

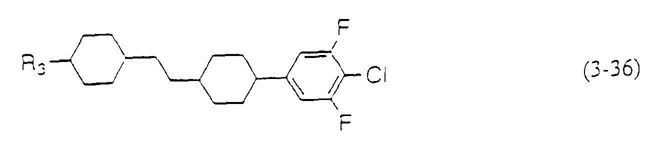

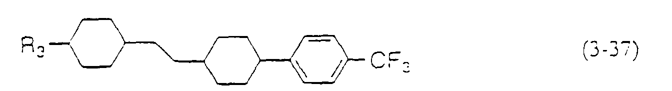

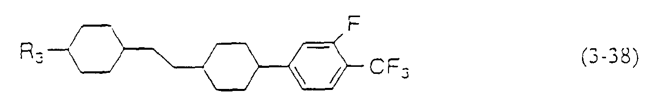

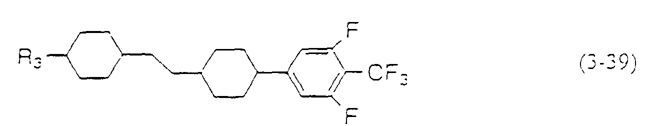

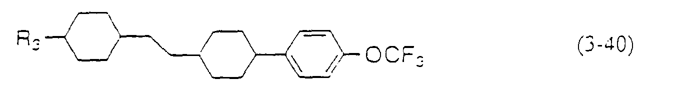

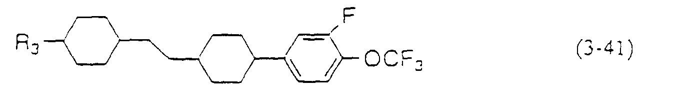

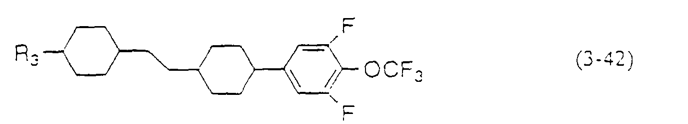

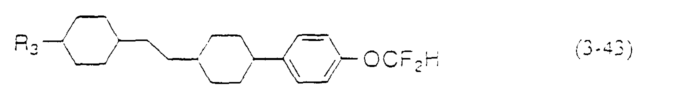

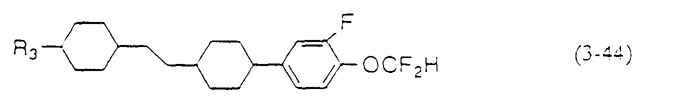

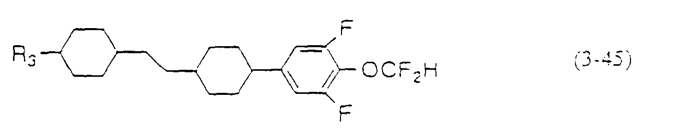

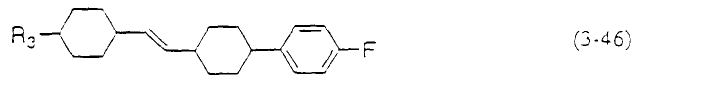

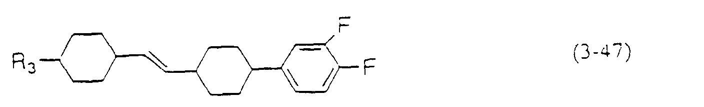

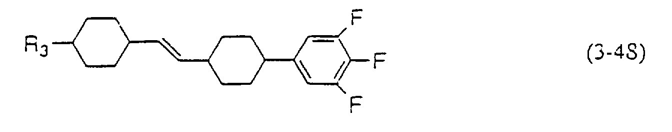

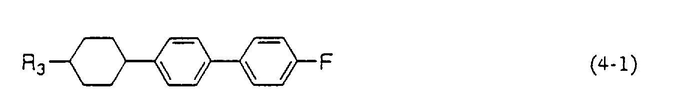

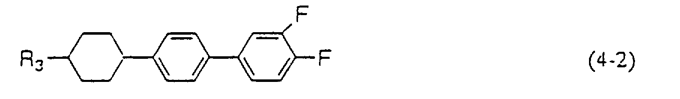

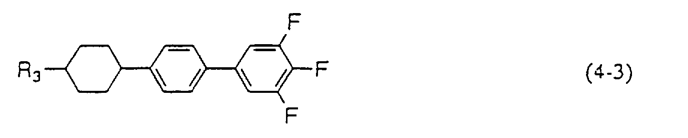

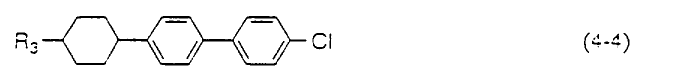

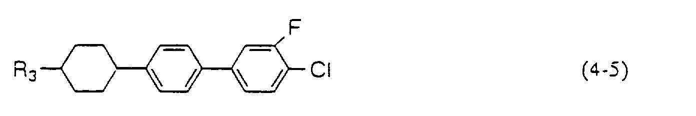

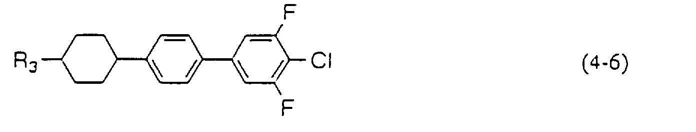

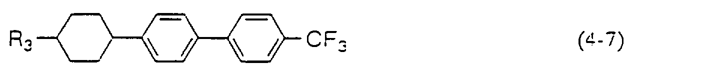

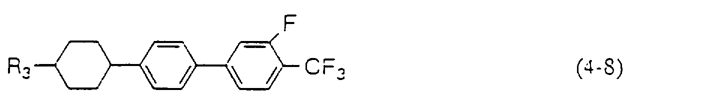

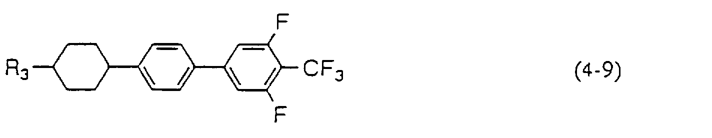

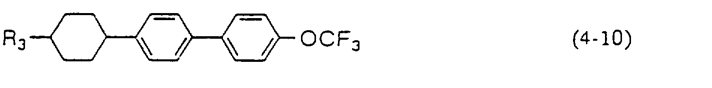

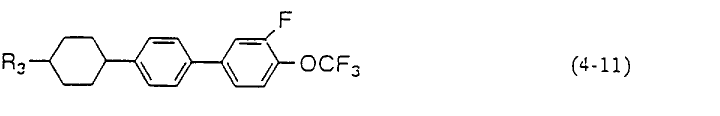

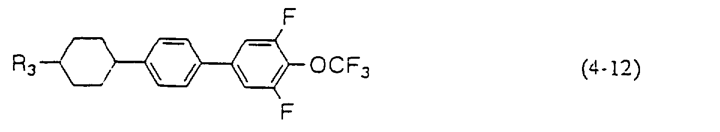

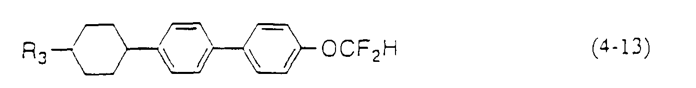

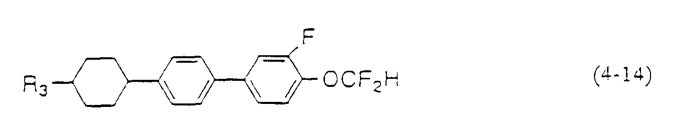

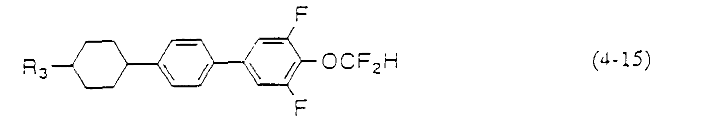

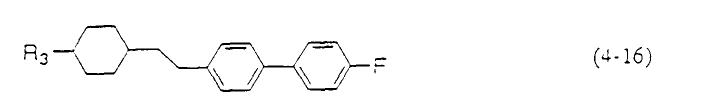

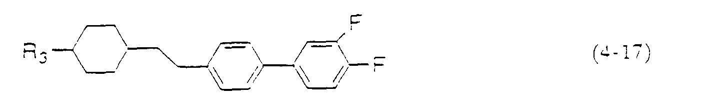

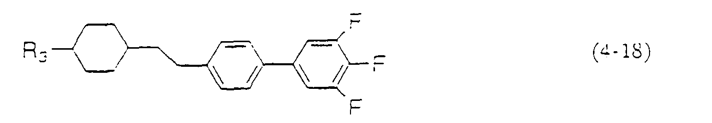

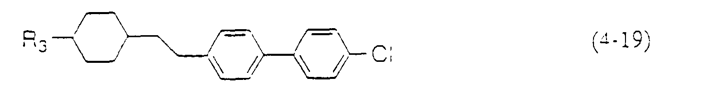

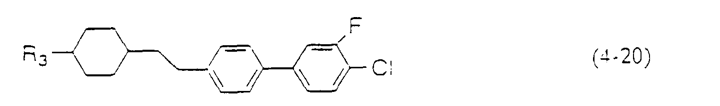

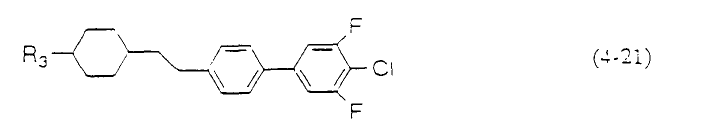

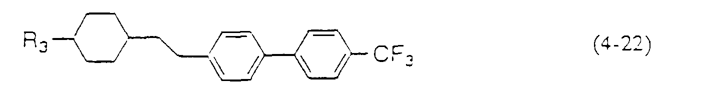

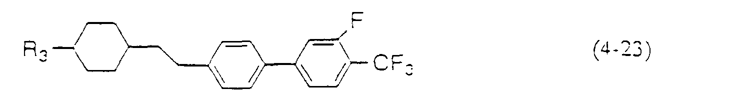

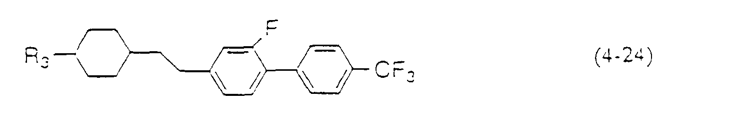

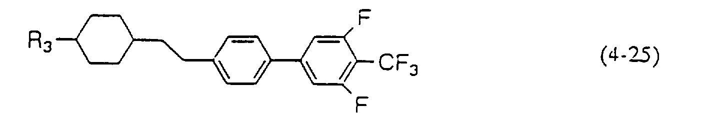

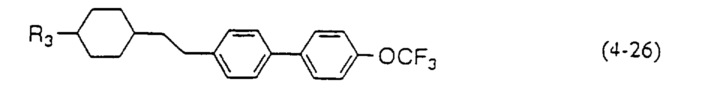

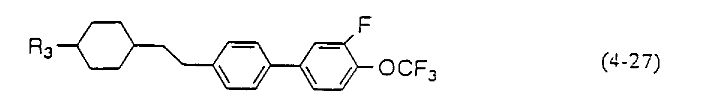

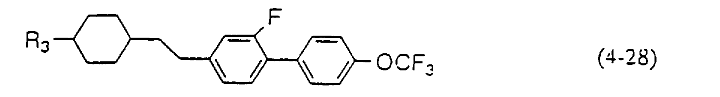

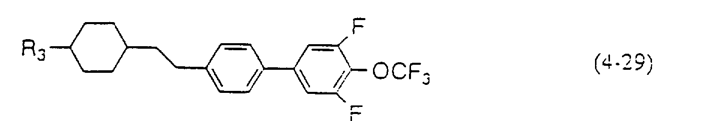

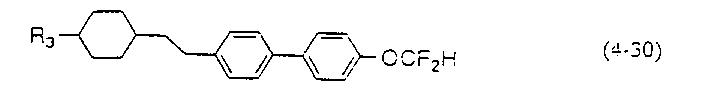

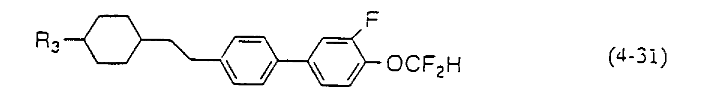

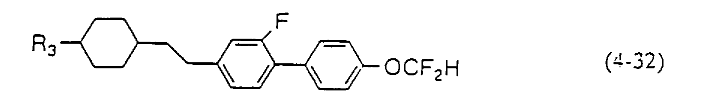

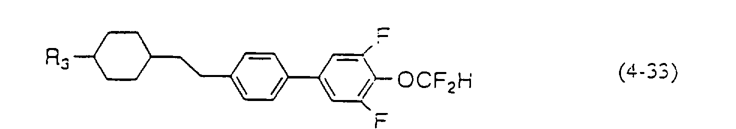

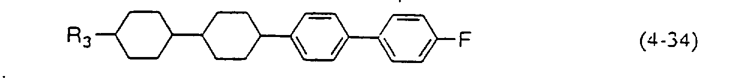

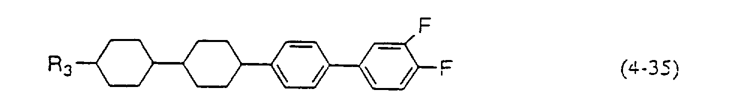

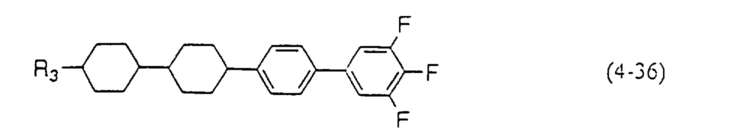

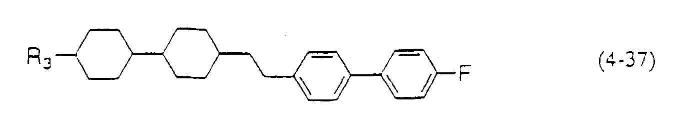

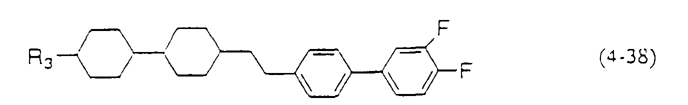

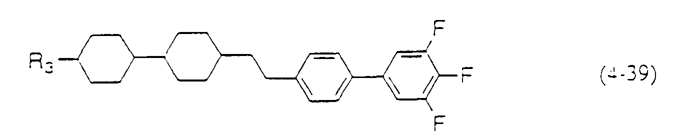

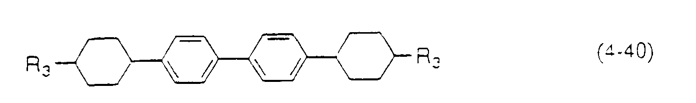

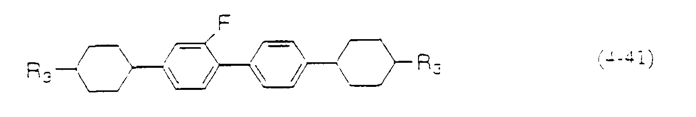

Compounds expressed by the general formulae (2) ∼ (4) are compounds with positive dielectric anisotropy value, which are very superior in thermal stability and chemical stability and which are necessary compounds in the case to prepare liquid crystalline compositions particularly for TFT (AM-LCD), which in turn is required high reliability such as high voltage holding ratio or high specific resistant value.

Amounts of compounds expressed by the general formulae (2) ∼ (4) used may be optionally within a range of from 1 to 99% by weight ralative to the total weight of the liquid crystalline compositions in the case to prepare liquid crystalline compositons for TFT, but from 10 to 97% by weight is preferable. More preferably, from 40 to 95% by weight is preferable. Furthermore, in such cases, some compounds expressed by the general formulae (5) ∼ (9) may be contained. Compounds expressed by the general formulae (2) ∼ (4) may be used in the case to prepare liquid crystalline compositions for STN display methods or conventional TN display methods.

As compounds expressed by the general formulae (5) ∼ (7) according to the invention, the following compounds may be preferably mentioned.
(R₄, R₅ and R₆ have the same meanings as above ones.)

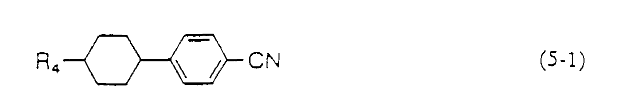

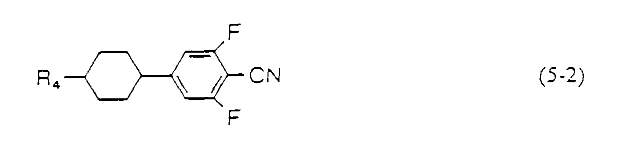

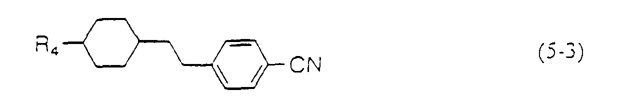

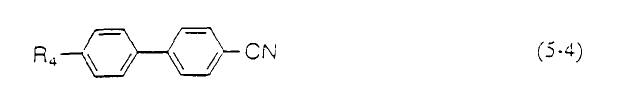

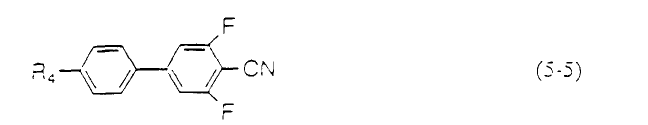

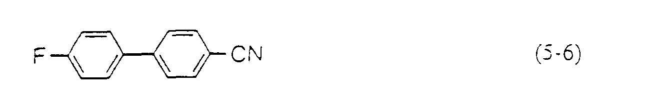

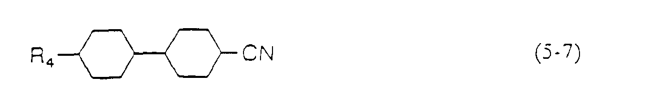

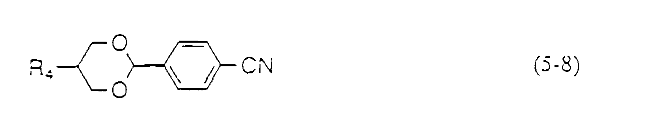

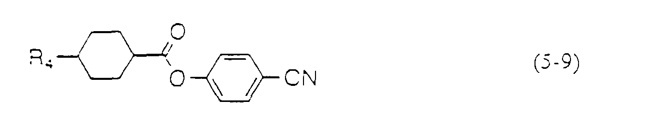

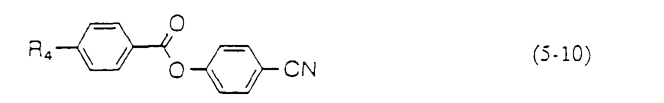

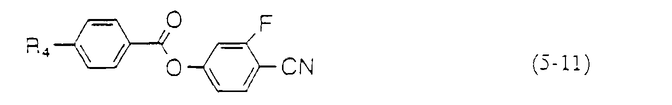

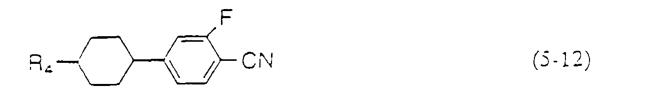

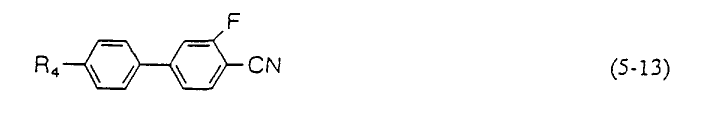

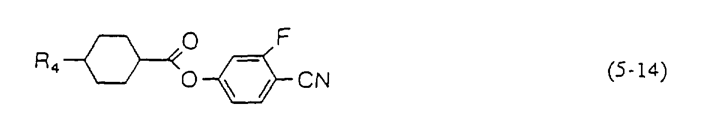

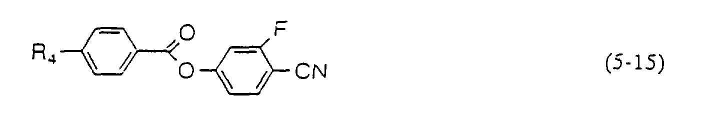

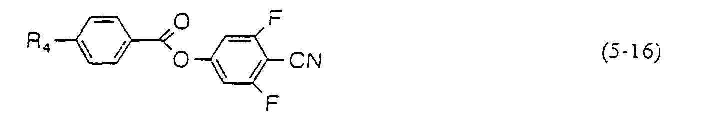

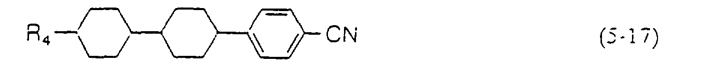

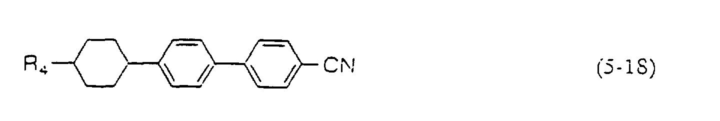

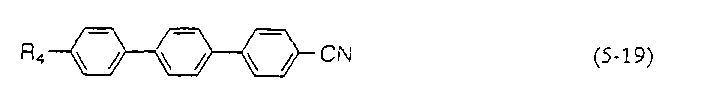

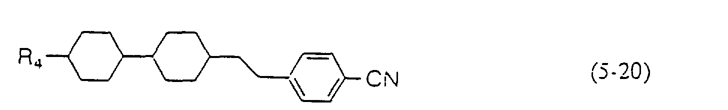

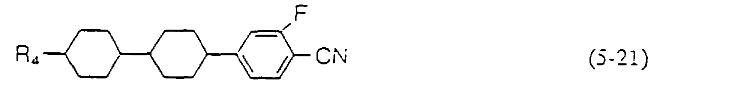

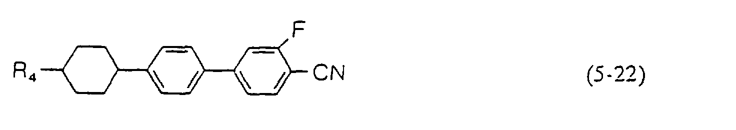

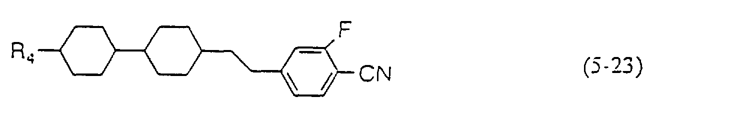

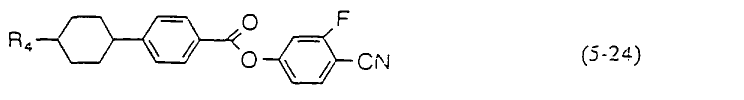

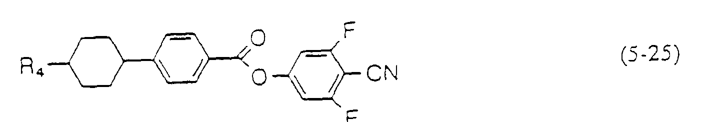

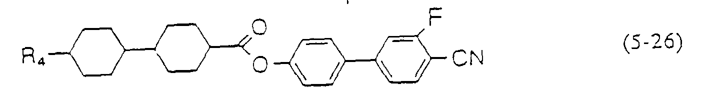

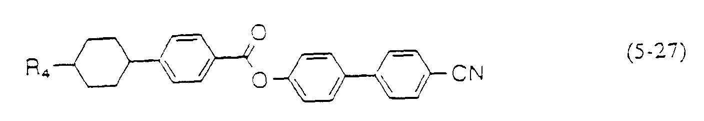

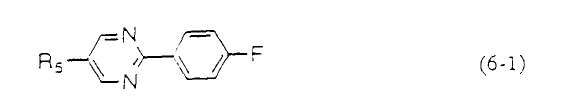

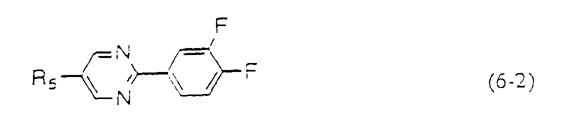

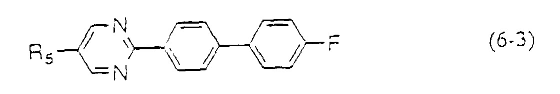

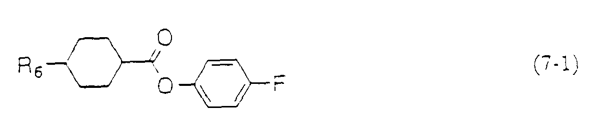

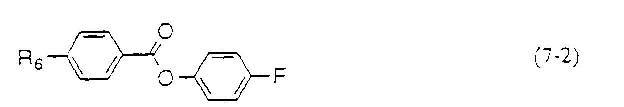

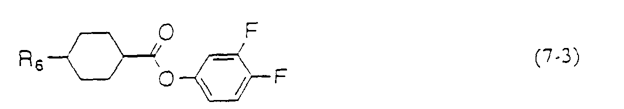

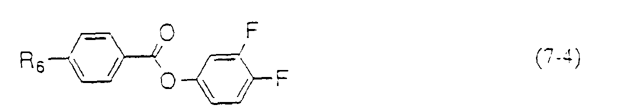

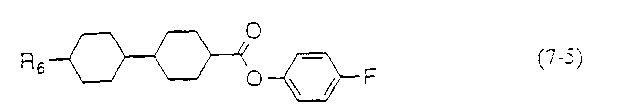

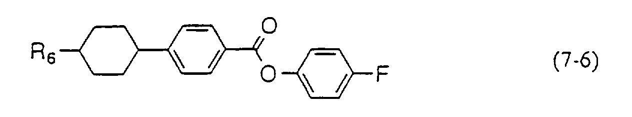

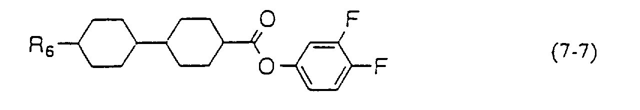

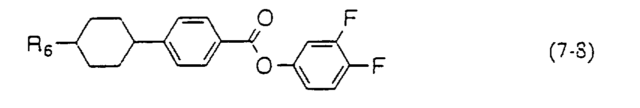

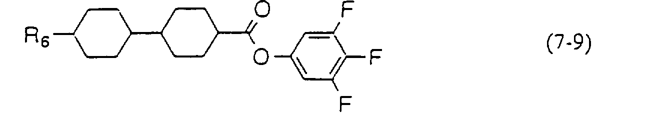

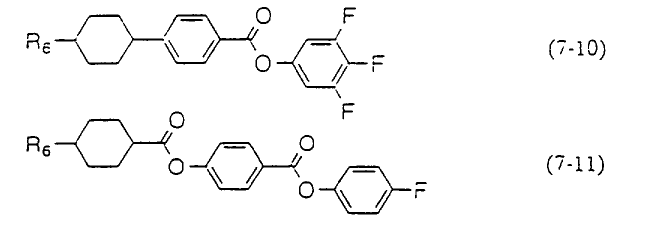

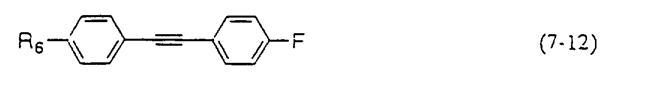

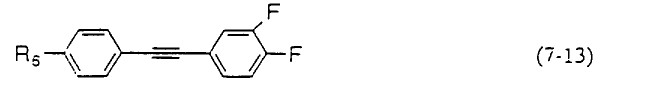

Compounds expressed by the general formulae (5) ∼ (7) have positive and high dielectric anisotropy values, and they are used particularly for an object to lower threshold voltages. Furtheremore, they are used for objects to enlarge nematic ranges such as control of viscosity, control of optical index anisotropy value and raise of clearing point etc.
Furthermore, they are used for an object to improve sharpness of threshold voltage.

As compounds expressed by the general formulae (8) and (9) according to the invention, the following compounds may be preferably mentioned.
(R₇, R₈, R₉ and R₁₀ have the same meanings as above ones.)

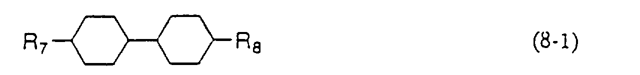

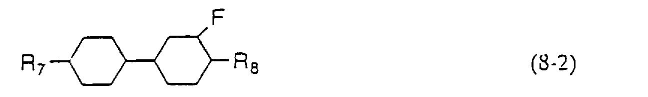

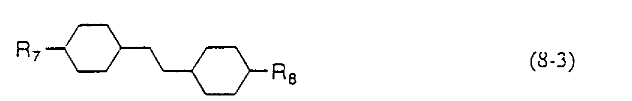

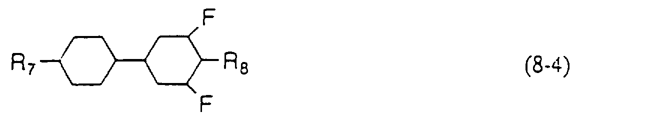

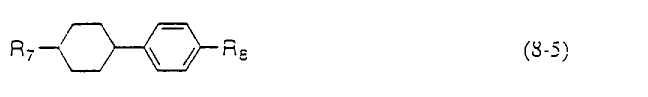

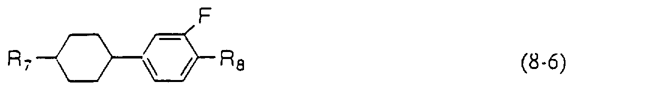

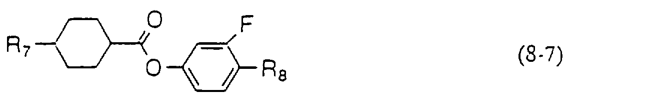

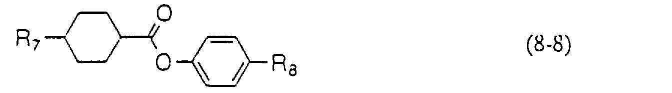

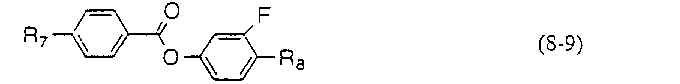

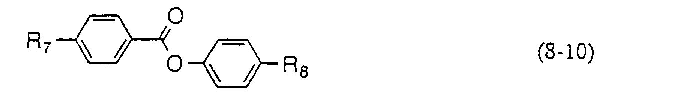

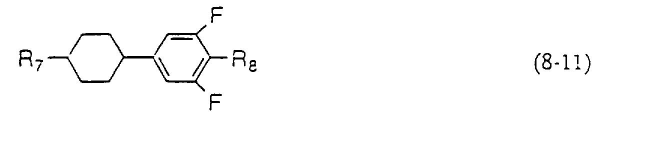

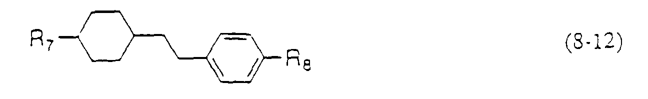

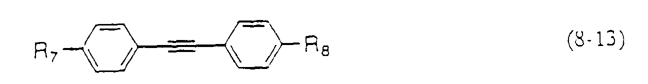

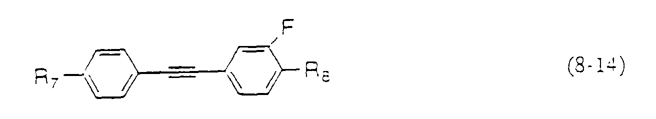

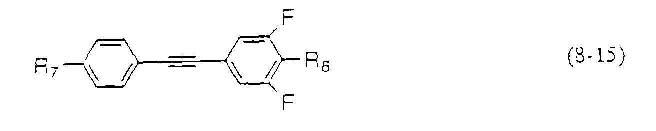

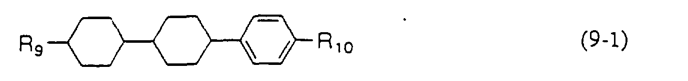

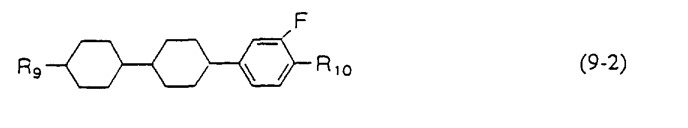

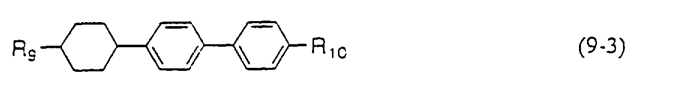

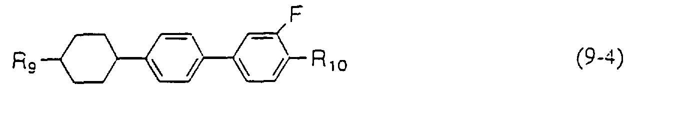

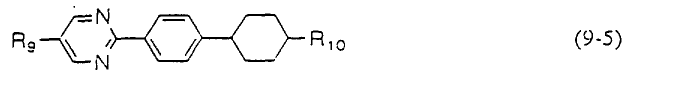

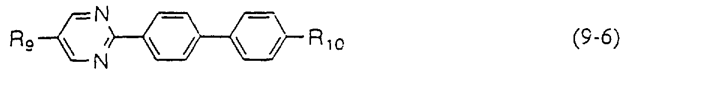

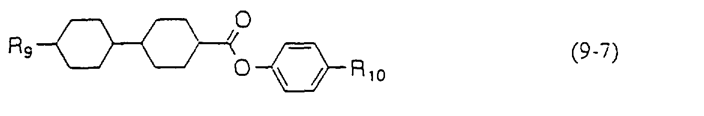

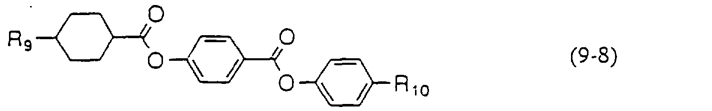

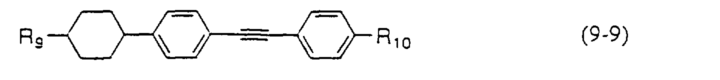

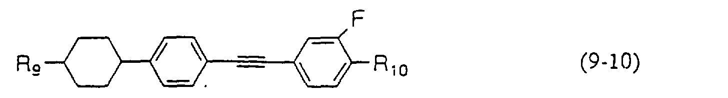

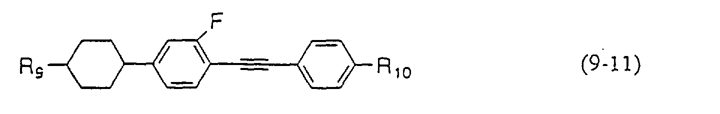

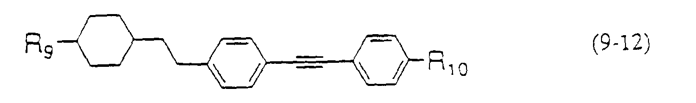

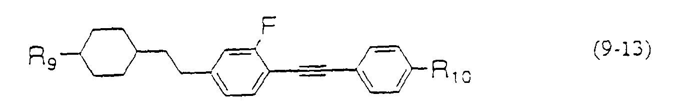

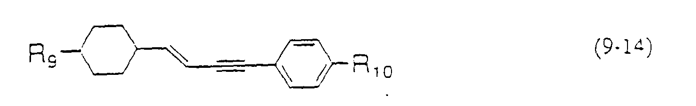

Compounds expressed by the general formulae (8) and (9) are compounds having negative or low dielectric anisotropy values. Compounds of the general formula (8) are used for an object to lower viscosity and/or to control refractive index anisotropy value. Furtheremore, compounds of the general formula (8) are used for an object to enlarge nematic ranges such as raise of clearing point etc. and for an object to control refractive index anisotropy value.

Compounds expressed by the general formulae (5) ∼ (9) are necessary compounds in the case to prepare liquid crystalline compositions particularly for STN display methods or conventional TN display methods.

Amounts of compounds expressed by the general formulae (5) ∼ (9) used may be optionally within a range of from 1 to 99% by weight ralative to the total weight of the liquid crystalline compositions in the case to prepare liquid crystalline compositons for conventinal TN display methods and STN display methods, but from 10 to 97% by weight is preferable. More preferably, from 40 to 95% by weight is preferable. Furthermore, in such cases, some compounds expressed by the general formulae (2) ∼ (4) may be contained.

Sharpness and angle range of vision can be improved by using liquid crystalline compositions according to the invention for TFT liquid crystalline display elements. Furthermore, since compounds of the formula (1) are low viscous compounds, response speeds of liquid crystalline display elements in which the said comopunds being used may be improved.

Liquid crystalline compositions used according to the invention are prepared by conventional methods per se. In general, methods for dissolving various components each other at high temperatures are taken. Furthermore, liquid crystalline materials of the invention may be improved according to intended applications by means of suitable additives and optimized. Those additives are known by the skilled men in the art and described in detail in literatures etc. Generally, chiral doping agents etc. are added in order to induce spiral structures of liquid crystals and to control necessary twist angles as well as to prevent opposite twists.

Furthermore, liquid crystalline compositions may be used as liquid crystalline compositions for guest-host (GH) modes by adding dichromatic dyestuffs such as merocyanine type, styryl type, azo type, azomethine type, azoxy type, quinophthalone type, anthraquinone type and tetrazine type ones etc. Alternatively, they may be used as liquid crystalline compositions for polymer dispersion type liquid crystalline display elements (PDLCD) represented by NCAP which is prepared by microcapsulating nematic liquid crystals or a polymer network liquid crystalline display element (PNLCD) in which three-dimentional network polymer is made in liquid crystals. Additionally, they may be used as liquid crystalline compositions for effective control birefringence (ECB) modes or dynamic scattering (DS) modes.

As nematic liquid crystalline compositions containing compounds according to the invention prepared as described above, the following examples of compositions may be shown. Wherein, the compounds described in the composition examples are shown according to the rules of the following tables.

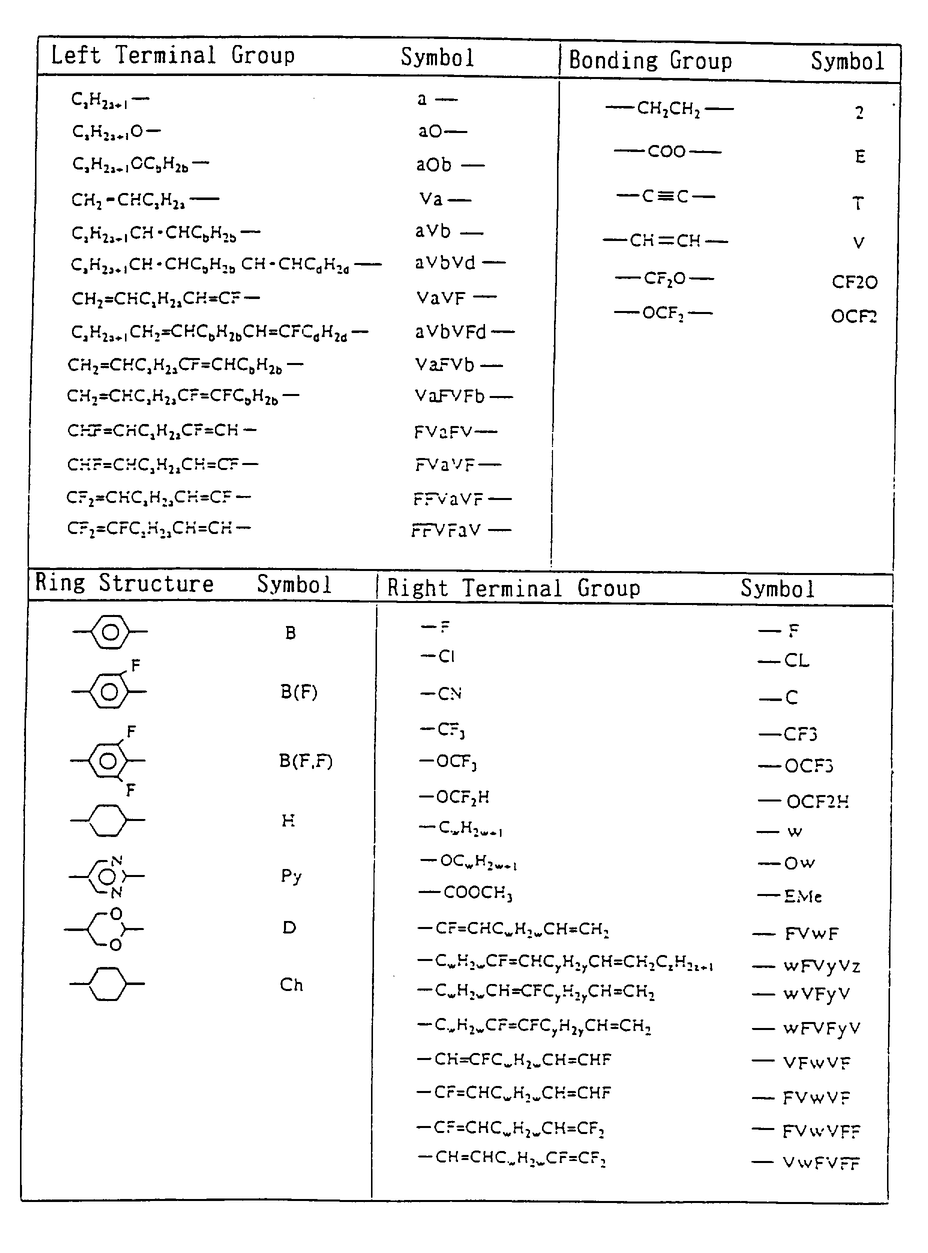

| Composition Example 1 | |
|---|---|
| V2VF-HB-C | 10.0% |
| V2VF-HHB-C | 5.0% |
| V2FVF-HHB(F)-C | 5.0% |
| V2VF-HEBB(F,F)-C | 5.0% |
| V2VF-HH-3 | 3.0% |
| FFVF2V-HH-3 | 3.0% |
| V2V-HH-FV2V | 4.0% |
| V2VF-HB-1 | 10.0% |
| FV2FV-HB-3 | 10.0% |
| V1VF-HHB-2VF2V | 10.0% |
| V2VF-HBTB-3 | 10.0% |
| V2VF-HVHB-3 | 10.0% |
| V2VF-HBOCF2B-3 | 10.0% |
| 3-HB(F)BH-VF2V | 5.0% |

| Composition Example 2 | |
|---|---|
| V2VF-HB-C | 20.0% |
| V2FVF-HHB(F)-C | 5.0% |
| V1VF-HHB-2VF2V | 5.0% |
| V2VF-HBTB-3 | 3.0% |
| V2VF-HBOCF2B-3 | 7.0% |
| | |
| V2-HB-C | 5.0% |
| 1V2-HB-C | 5.0% |
| 3-HB-C | 16.0% |
| 5-HB-C | 7.0% |
| 3-HB(F)-C | 8.0% |
| 2-BEB-C | 3.0% |
| V2-HHB-1 | 4.0% |
| 3-HHB-O1 | 3.0% |
| 3-HHB-3 | 3.0% |
| 3-H2BTB-2 | 2.0% |
| 3-H2BTB-3 | 2.0% |
| 3-H2BTB-4 | 2.0% |

| Composition Example 3 | |
|---|---|
| V2VF-HEBB(F,F)-C | 5.0% |
| V2FVF-HHB(F)-C | 10.0% |
| | |
| 1V2-BEB(F,F)-C | 11.0% |
| 2O1-BEB(F)-C | 5.0% |
| 3O1-BEB(F)-C | 9.0% |
| 3-HB(F)-C | 15.0% |
| 3-HH-4 | 3.0% |
| 1O1-HH-3 | 3.0% |
| 4-BTB-O2 | 5.0% |
| 2-HHB(F)-C | 8.0% |
| 3-HHB(F)-C | 4.0% |
| 3-H2BTB-2 | 4.0% |
| 3-H2BTB-3 | 4.0% |
| 3-H2BTB-4 | 4.0% |
| 2-BTB-1 | 1.0% |
| 1-BTB-6 | 2.0% |
| 4-BTB-4 | 1.0% |
| 3-HH-2V | 3.0% |
| 4-HH-V | 3.0% |

| Composition Example 4 | |
|---|---|
| V1VF-HHB-2VF2V | 8.0% |
| V2VF-HB-C | 5.0% |
| | |
| 2-BB-C | 5.0% |
| 2O2O-BB-C | 4.0% |
| 1O1-HB-C | 5.0% |
| 2O1-HB-C | 7.0% |
| 2-BEB-C | 12.0% |
| 5-PyB-F | 8.0% |
| 2-PyB-2 | 2.0% |
| 3-PyB-2 | 2.0% |
| 4-PyB-2 | 2.0% |
| V-HHB-1 | 5.0% |
| 3-HHB-1 | 7.0% |
| 3-HHB-3 | 5.0% |
| 2-PyBH-3 | 4.0% |
| 3-PyBH-3 | 4.0% |
| 4-PyBH-3 | 4.0% |
| 3-PyBB-F | 4.0% |
| 4-PyBB-F | 4.0% |
| 6-PyBB-2 | 3.0% |

| Composition Example 5 | |
|---|---|
| 3-HB(F) BH-VF2V | 3.0% |
| | |
| 3-PyB(F)-F | 6.0% |
| 3O2O-BEB-C | 4.0% |
| 3-BEB-C | 12.0% |
| 3-DB-C | 10.0% |
| 4-DB-C | 10.0% |
| 3-HEB-O4 | 8.0% |
| 4-HEB-O2 | 6.0% |
| 5-HEB-O1 | 6.0% |
| 3-HEB-O2 | 5.0% |
| 5-HEB-O2 | 4.0% |
| 3-HHB-1 | 6.0% |
| 3-HHEBB-C | 3.0% |
| 3-HBEBB-C | 3.0% |
| 1O-BEB-2 | 5.0% |
| 4-HEB-3 | 3.0% |
| 5-HEB-1 | 3.0% |
| 6-PyB-O2 | 3.0% |

| Composition Example 6 | |
|---|---|
| V2VF-HBTB-3 | 4.0% |
| V2VF-HVHB-3 | 3.0% |
| | |
| 3-HB-C | 20.0% |
| 3-HHB-1 | 7.0% |
| 3-HHB-3 | 8.0% |
| 5-HEB-F | 3.0% |
| 7-HEB-F | 3.0% |
| 3-HHEB-F | 1.0% |
| 5-HHEB-F | 1.0% |
| 3-HEB-O4 | 4.0% |
| 4-HEB-O2 | 3.0% |
| 5-HEB-O1 | 3.0% |
| 3-HEB-O2 | 2.5% |
| 5-HEB-O2 | 2.0% |
| 3-HB(F)TB-2 | 4.0% |
| 3-HB(F)TB-3 | 4.0% |
| 3-HB(F)VB-4 | 3.0% |
| 3-H2BTB-2 | 3.0% |
| 3-H2BTB-3 | 3.0% |
| 3-H2BTB-4 | 3.0% |
| 3-HHEBB-C | 2.0% |
| 5-HHEBB-C | 2.0% |
| 3-HBEBB-C | 3.0% |
| 5-HBEBB-C | 3.0% |
| 3-HEBEB-F | 3.0% |
| 3-HH-COOMe | 2.5% |
| 1O1-HBBH-3 | 2.0% |

| Composition Example 7 | |
|---|---|
| V2VF-HHB-C | 6.0% |
| | |
| 5-PyB(F)-F | 13.0% |
| 2-HB(F)-C | 10.0% |
| 3-HB(F)-C | 12.0% |
| 3O-BB-C | 8.0% |
| 2-HHB-C | 6.0% |
| 3-HHB-C | 6.0% |
| 4-HHB-C | 6.0% |
| 2-HHB(F)-C | 5.0% |
| 3-HHB (F) -C | 5.0% |
| 3-PyBB-F | 7.0% |
| 4-PyBB-F | 6.0% |
| 5-HBB-C | 5.0% |
| 3-HB(F)EB(F)-C | 5.0% |

| Composition Example 8 | |
|---|---|
| FFV2VF-HBB(F)-OCF | 5.0% |
| V2VF-HBOCF2B-3 | 5.0% |
| | |
| 5-H2B(F)-F | 4.0% |
| 7-HB(F)-F | 10.0% |
| 2-HHB(F)-F | 12.0% |
| 3-HHB(F)-F | 12.0% |
| 5-HHB(F)-F | 12.0% |
| 2-H2HB(F)-F | 12.0% |
| 3-H2HB(F)-F | 6.0% |
| 5-H2HB(F)-F | 12.0% |
| 2-HBB(F)-F | 2.5% |
| 3-HBB(F)-F | 2.5% |
| 5-HBB(F)-F | 5.0% |

| Composition Example 9 | |
|---|---|
| FFV2VF-HBB(F)-OCF3 | 8.0% |
| | |
| 7-HB(F,F)-F | 5.0% |
| 3-HBB(F,F)-F | 5.0% |
| 5-HBB(F,F)-F | 5.0% |
| 3-HBB(F,F)-F | 7.0% |
| 5-HBB(F,F)-F | 5.0% |
| 3-HH2B(F,F)-F | 8.0% |
| 5-HH2B(F,F)-F | 5.0% |
| 3-H2HB(F,F)-F | 10.0% |
| 4-H2HB(F,F)-F | 10.0% |
| 5-H2HB(F,F)-F | 10.0% |
| 3-HHEB(F,F)-F | 8.0% |
| 4-HHEB(F,F)-F | 3.0% |
| 5-HHEB(F,F)-F | 3.0% |
| 3-HBEB(F,F)-F | 2.0% |
| 5-HBEB(F,F)-F | 2.0% |
| 3-HHHB(F,F)-F | 2.0% |
| 5-HH2BB(F,F)-F | 2.0% |

| Composition Example 10 | |
|---|---|
| FFVF2V-HH-3 | 3.0% |
| V2VF-HVHB-3 | 4.0% |
| | |
| 7-HB(F,F)-F | 2.0% |
| 2-HHB(F)-F | 10.0% |
| 3-HHB(F)-F | 14.0% |
| 5-HHB(F)-F | 14.0% |
| 2-H2HB(F)-F | 4.0% |
| 3-H2HB(F)-F | 2.0% |
| 5-H2HB(F)-F | 4.0% |
| 3-HHB(F,F)-F | 8.0% |
| 4-HHB(F,F)-F | 4.0% |
| 3-H2HB(F,F)-F | 6.0% |
| 4-H2HB(F,F)-F | 5.0% |
| 5-H2HB(F,F)-F | 5.0% |
| 3-HH2B(F,F)-F | 8.0% |
| 5-HH2B(F,F)-F | 7.0% |

| Composition Example 11 | |
|---|---|
| FV2FV-HB-3 | 5.0% |
| V2VF-HB-1 | 5.0% |
| | |
| 5-HB-F | 2.0% |
| 7-HB(F)-F | 3.0% |
| 2-HHB(F)-F | 14.0% |
| 3-HHB(F)-F | 14.0% |
| 5-HHB(F)-F | 14.0% |
| 3-HB-O2 | 5.0% |
| 3-HHB-F | 4.0% |
| 3-HHB-1 | 6.0% |
| 3-HHB-3 | 6.0% |
| 2-HBB-F | 6.0% |
| 3-HBB-F | 5.0% |
| 3-HHEB-F | 3.0% |
| 5-HHEB-F | 3.0% |
| 3-HBEB-F | 3.0% |
| 3-HHEBB-F | 2.0% |

| Composition Example 12 | |
|---|---|
| V2VF-HBTB-3 | 6.0% |
| | |
| 7-HB(F,F)-F | 7.0% |
| 3-HB-CL | 5.0% |
| 7-HB-CL | 5.0% |
| 2-BTB-O1 | 10.0% |
| 2-HBB(F)-F | 2.5% |
| 3-HBB(F)-F | 2.5% |
| 5-HBB(F)-F | 5.0% |
| 3-HBB(F,F)-F | 7.0% |
| 5-HBB(F,F)-F | 10.0% |
| 2-HHB-CL | 5.0% |
| 3-HHB-CL | 3.0% |
| 3-HB(F)TB-2 | 6.0% |
| 3-HB(F)TB-4 | 6.0% |
| 2-H2BTB-2 | 4.0% |
| 2-H2BTB-3 | 4.0% |
| 3-H2HB(F)-CL | 4.0% |
| 5-H2HB(F)-CL | 3.0% |
| 3-H2BB(F,F)-F | 3.0% |

| Composition Example 13 | |
|---|---|
| FFV2VF-HBB(F)-OCF3 | 10.0% |
| V2VF-HBOCF2B-3 | 5.0% |
| FV2FV-HB-3 | 10.0% |
| | |
| 5-HB-F | 10.0% |
| 6-HB-F | 5.0% |
| 7-HB-F | 5.0% |
| 2-HHB-OCF3 | 5.0% |
| 3-HHB-OCF3 | 5.0% |
| 5-HHB-OCF3 | 5.0% |
| 3-HH2B-OCF3 | 6.0% |
| 5-HH2B-OCF3 | 6.0% |
| 3-HB(F)B-3 | 4.0% |
| 5-HB(F)B-3 | 4.0% |
| 2-HBB(F)-F | 5.0% |
| 3-HBB(F)-F | 5.0% |
| 5-HBB(F)-F | 10.0% |

| Composition Example 14 | |
|---|---|
| FFV2VF-HBB(F)-OCF3 | 6.0% |
| V2VF-HVHB-3 | 6.0% |
| | |
| 5-HB-F | 3.0% |
| 6-HB-F | 3.0% |
| 7-HB-F | 3.0% |
| 3-HHB-OCHF2 | 4.0% |
| 5-HHB-OCHF2 | 4.0% |
| 3-HHB(F,F)-OCHF2 | 9.0% |
| 5-HHB(F,F)-OCHF2 | 9.0% |
| 2-HHB-OCF3 | 6.0% |
| 3-HHB-OCF3 | 6.0% |
| 4-HHB-OCF3 | 6.0% |
| 5-HHB-OCF3 | 6.0% |
| 3-HH2B(F)-F | 10.0% |
| 5-HH2B(F)-F | 10.0% |
| 3-HHEB(F)-F | 4.0% |
| 5-HHEB(F)-F | 5.0% |

| Composition Example 15 | |
|---|---|
| V2V-HH-FV2V | 5.0% |
| V2VF-HB-C | 5.0% |
| | |
| 4-HHEB(F)-F | 5.0% |
| 5-HHEB(F)-F | 5.0% |
| 2-BEB(F)-C | 5.0% |
| 3-BEB(F)-C | 7.0% |
| 4-BEB(F)-C | 5.0% |
| 5-BEB(F)-C | 7.0% |
| 103-HB(F)-C | 6.0% |
| 3-HHEB(F)-F | 5.0% |
| 5-HHEB(F)-F | 5.0% |
| 2-HBEB(F)-C | 5.0% |
| 3-HBEB(F)-C | 5.0% |
| 4-HBEB(F)-C | 5.0% |
| 5-HBEB(F)-C | 5.0% |
| 3-HBTB-2 | 10.0% |
| V2-HH-3 | 5.0% |
| V2-HHB-1 | 5.0% |

| Composition Example 16 | |
|---|---|
| V2VF-HB-C | 5.0% |
| V2FVF-HHB(F)-C | 5.0% |
| V2VF-HHB-C | 5.0% |
| V2V-HH-FV2V | 5.0% |
| | |
| V2-HB-C | 3.0% |
| 4-BB-2 | 5.0% |
| 3-BB-C | 5.0% |
| 5-BB-C | 5.0% |
| 2-HB(F)-C | 5.0% |
| 3-H2B-O2 | 5.0% |
| 5-H2B-O3 | 10.0% |
| 3-BEB-C | 5.0% |
| 5-HEB-O1 | 6.0% |
| 5-HEB-O3 | 6.0% |
| 5-BBB-C | 3.0% |
| 4-BPyB-C | 3.0% |
| 4-BPyB-5 | 3.0% |
| 5-HB2B-4 | 4.0% |
| 5-HBB2B-3 | 4.0% |
| V2-HH-1O1 | 3.0% |
| 1V2-HBB-3 | 5.0% |

Compounds (1) according to the invention can be prepared by utilizing general organic synthetic chemical methods. That is, known reactions described in books and magazines such as Novel Experimental Chemical Courses. Organic Synthesis and Organic Reactions etc. may be combined to carry out synthesis. 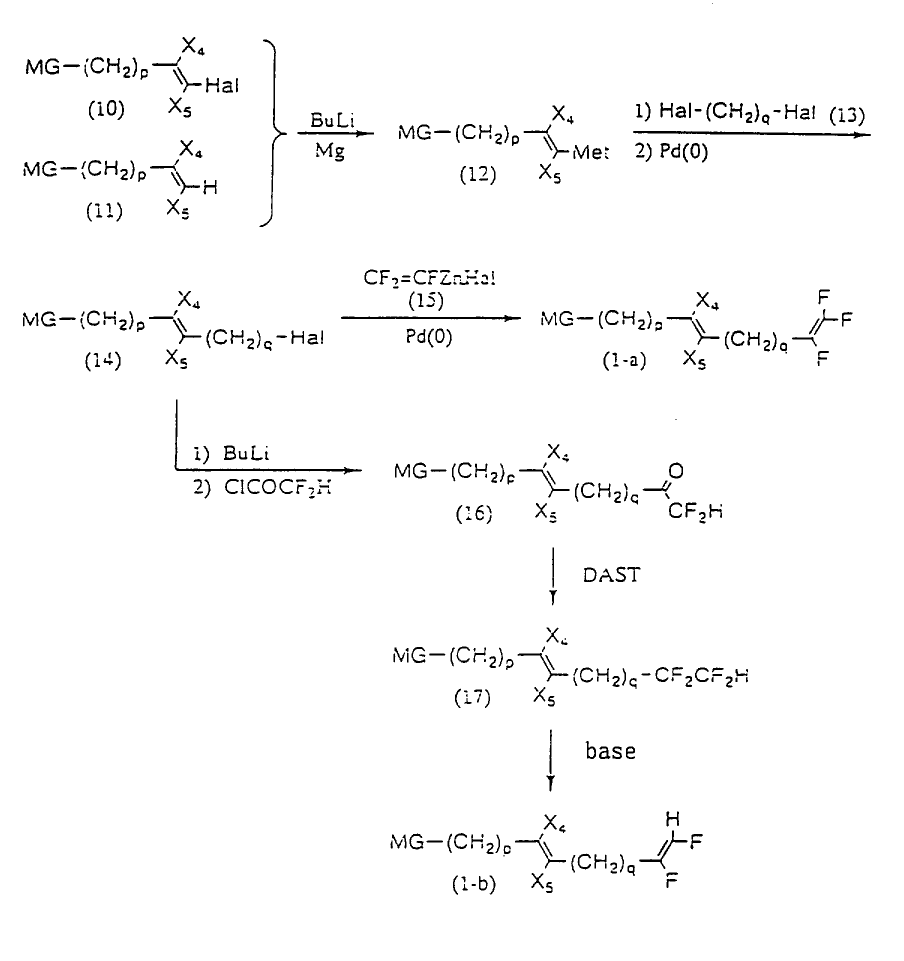 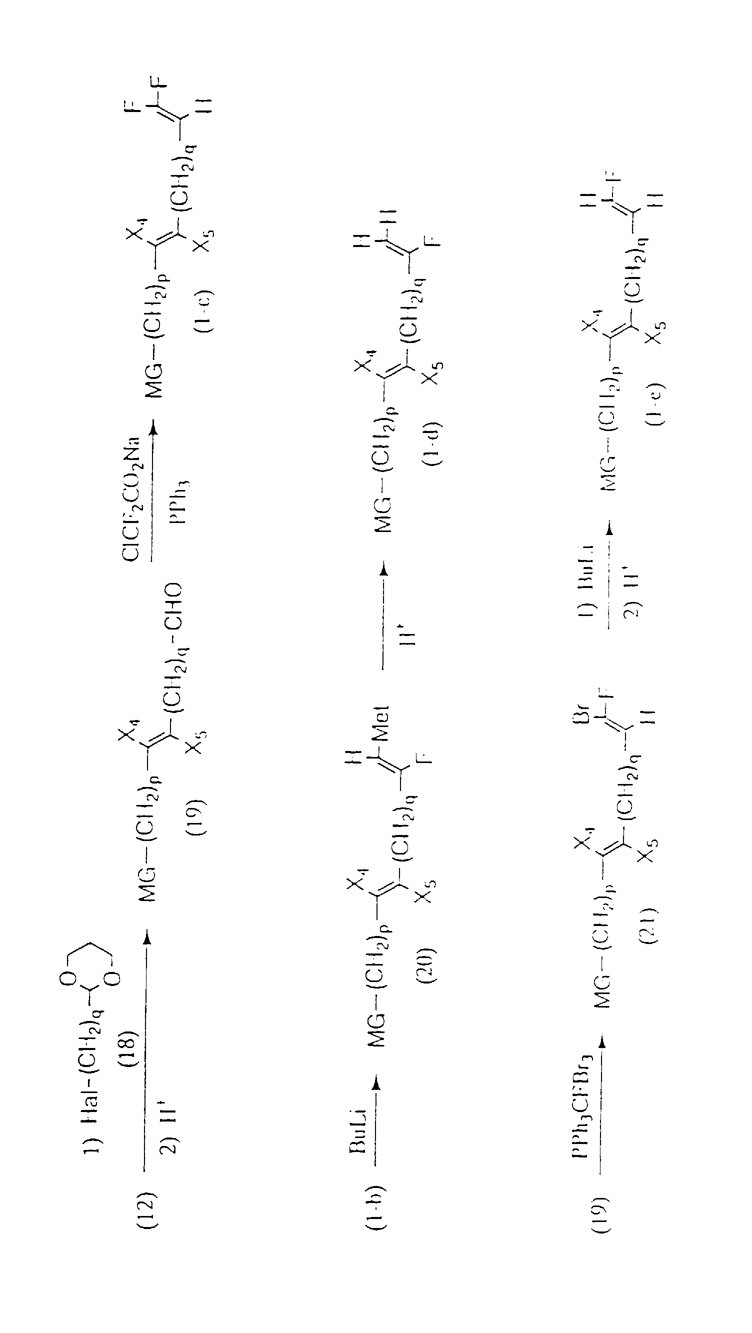 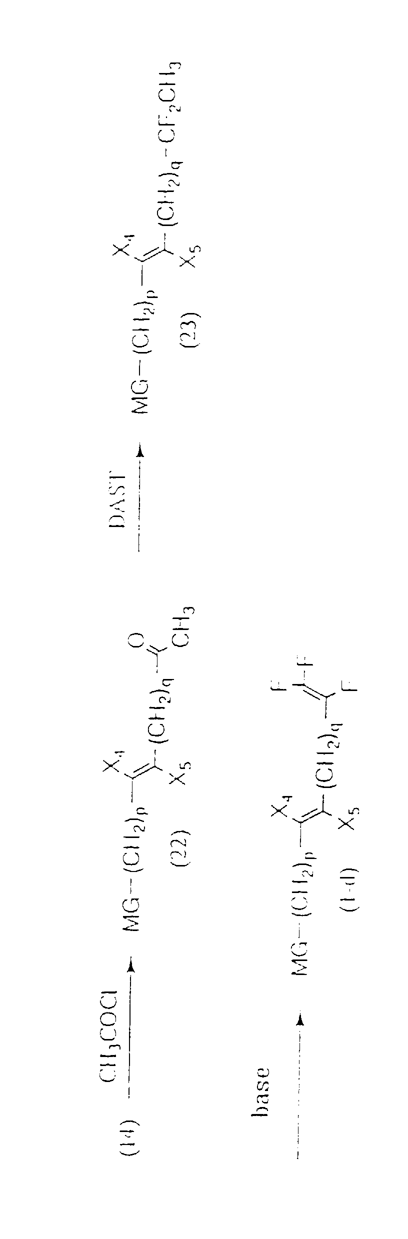 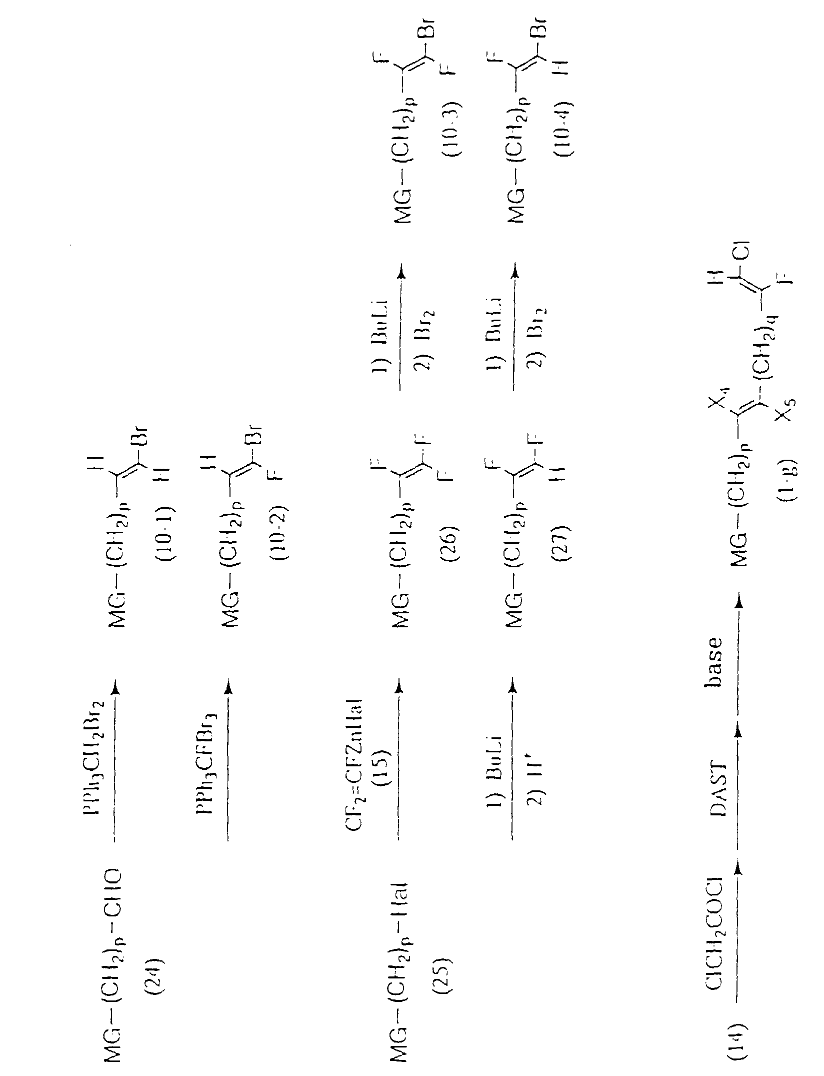 (In the above formulae, MG denotes a methogen, i.e. a central backbone constituted by a ring, Hal denotes a bromine atom or an iodine atom, Met denotes Li or MgBr, and X₄, X₅, p and q denote the same meanings as above ones.)

That is, alkyl lithium or magnesium is reacted with a halogenated alkenyl derivative (10) or an alkenyl derivative (11), to obtain an organometallic compound (12). Then, α,ω-dihalogenoalkane (13) is reacted with it for carrying out a cross-coupling reaction, to obatin (14). By reacting (14) with a zinc compound (15) prepared from zinc and halogenataed trifluoroethene, there can be prepared a compound (1-a), that is a compound of formula (1) in which all of X₆, X₇ and X₈ being fluorine atoms.

After treating (14) with alkyl lithium, difluoroacetic chloride is reacted with it to make a ketone derivative (16), and thereafter a fluorinating agent such as DAST etc. is reacted with it for fluorination of a carbonyl group, to prepare (17). By treating (17) with a base, there can be prepared a compound (1-b), that is, a compound of formula (1) in which X₆ and X₈ being fluorine atoms and X₇ being a hydrogen atom.

By reacting a dioxane derivative such as (18) or a dioxolane derivative with (12) for carrying out a cross-coupling reaction and thereafter carrying out an open-ring reaction of the treated dioxane ring or dioxolane ring under an acidic condition, there can be prepared an aldehyde derivative (19). By reacting triphenyl phosphine and sodium chlorodifluoroacetate with (19), there can be prepared a compound (1-c), that is a compound of formula (1) in which X₆ being a hydrogen atom and X₇ and X₈ being fluorine atoms.

By reacting alkyl lithium with (1-b), the preparing method of which is already shown above, then substituting a terminal fluorine atom by metal such as lithium etc. and thereafter treating with a protic solvent, there can be prepared a compound (1-d), that is a compound of formula (1) in which X₆ being a fluorine atom and X₇ and X₈ being hydrogen atoms.

By reacting alkyl lithium with (21) which may be prepared by the reaction of (19) and a phosphonium salt of fluorotribromomethane and then treating it under the above-mentioned condition, there can be prepared a compound (1-e), that is a compound of formula (1) in which X₆ and X₇ being hydrogen atoms and X₈ being a fluorine atom.

By treating (23) which is obtained by fluorination of a carbonyl comopund (22), which is in turn prepared in a cross-coupling reaction of (14) and acetic chloride, under a basic condition to carry out dehydrofluorination, there can be prepared a compound (1-f), that is a compound of formula (1) in which X₆ being a fluorine atom and X₇ and X₈ being hydrogen atoms.

Furthermore, by reacting a phosphonium salt of methylbromide with aldehyde (19), there can be easily prepared a compound in which all of X₆, X₇ and X₈ being hydrogen atoms.

The above-mentioned halogenated alkenyl comopund (10) can be preferably prepared by the following methods.

That is, by reacting a phosphonium salt of methylbromide with (24) which may be synthesized by any known method from literatures, there can be prepared (10-1) in which X₄ and X₅ being hydrogen atoms.

By reacting a phosphonium salt of fluorotribromomethane with (24), there can be prepared (10-2) in which X₄ being a hydrogen atom and X₅ being a fluorine atom.

By carrying out lithiation of one terminal fluorine atom of (26), which is obtained by the reaction of halide (25) prepared by any known method from literatures and (15), under the same condition as the above one and treating it with a halogen, there can be prepared (10-3) in which both of X₄ and X₅ being fluorine atoms.

By carrying out lithiation, treatment with a protic solvent, again lithiation with (26), and then treatment with halogen, (10-4) can be prepared.

By carrying out lithiation of halogen sites in the above-mentioned (10-1) ∼ (10-4) and treatment with a protic solvent, (11) can be prepared.

Furthermore, chlorides can be prepared similar to the above-mentioned methods. For example, by reacting chloroacetic chloride with (14) according to the above-mentioned method, chlorides such as (1-g) can be prepared.

### Examples

The invention will be illustrated in more detail by the following examples.

### Example 1

Preparation of 4-(4-(4-(1,2-difluoro-1,5-hexadienyllcyclohexyl)cyclohexyl)-1-pentylbenzene 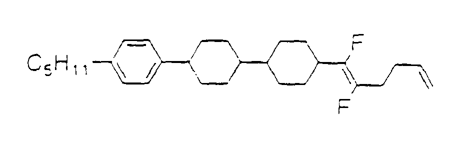 (Compound No. 1, a compound according to the formula (1) wherein R₁ is a pentyl group, X₂ and X₃ are covalent bonds, C and D are 1,4-cyclohexane rings, B is a 1,4-phenylene ring, m=0, n=1, p is 0, q is 2, X₄ and X₅ are fluorine atoms, and X₆, X₇ and X₈ are hydrogen atoms).

A corresponding zinc compound (correspoding to 0.10 mol) prepared from iodotrifluoroethene and zinc powders in a solution of THF was added to a mixture of 4-(4-(4-iodocyclohexyl)cyclohexyl)-1-pentylbenzene (0.05 mol), tetrakis(triphenyl)phosphine)palladium (0) (0.005 mol) and 70ml of THF at -30°C, stirred for 30 minutes, thereafter returned to the room temperature gradually, and heated under reflux for 1 hour. They were cooled again to below 0°C, to which 20ml of an aqueous saturated ammonium chloride solution was added and then returned to the room temperature with stirring. Extraction with 150ml of heptane was carried out, and an organic layer was washed with water for three times and dried with anhydrous magnesium sulfate. The solvent was distilled off under a decreased pressure and the residue was recrystallized from 75ml of ethanol, to obtain 4-(4-(4-(trifluoroethenyl)cyclohexyl)cyclohexyl)-1-pentylbenzene (0.042 mol).

45ml of a solution of 4-(4-(4-(trifluoroethenyl)cyclohexyl)yclohexyl)-1-pentylbenzene (0.04 mol) in THF was cooled to -78 °C, to which butyl lithium (corresponding to 0.04 mol) was added dropwise and stirred for 1 hour at the same temperature. Bis(triphenylphosphine)palladium(II) dichloride (0.004 mol) was added, then 50ml of a solution of 2-(2-bromoethyl)-1,3-dioxane (0.044 mol) in THF was added with maintaining the same temperature, and stirred for 1 hour. 20ml of an aqueous saturated ammonium chloride solution was added, returned to the room temparature gradually, and thereafter extracted with 100ml of toluene. After washing an organic layer with water twice, toluene was distilled off under a decreased pressure, to obtain crude 4-(4-(4-(1,2-difluoro-4-(1,3-dioxy-2-yl)-1-butene)cyclohexyl)cyclohexyl)-1-pentylbenzene.

A mixture of the prepared compound (unpurified), 10ml of 95% formic acid and 100ml of toluene was heated under reflux for 1 hour and allowed to cool to the room temperature. The reactant was transferred to a separatory funnel and an organic layer was washed with water twice, washed with a saturated sodium hydrogen carbonate solution twice, and dried with anhydrous magnesium sulfate. The solvent was distilled off under a decreased pressure and the residue was treated with a column chromatography (eluate: toluene), to obtain 4-(4-(4-(1,2-difluoro-5-oxo-1-pentenyl)-cyclohexyl)cyclohexyl)-1-pentylbenzene (0.021 mol).

Potasium-t-butoxide (0.031 mol) was added to a mixture of triphenyl phosphonium methane iodide (0.03 mol) and 50ml of THF and stirred for 20 minutes, to obtain a red solution. To the said solution, 20ml of a solution of 4-(4-(4-(1,2-difluoro-5-oxo-1-pentenyl)-cyclohexyl)cyclohexyl)-1-pentylbenzene (0.02 mol) in THF was added dropwise at below 10°C and stirred for 1 hour. To the reactant, 50ml of water and 100ml of heptane were added to carry out extraction.

An organic layer was washed with water for three times and dried with anhydrous magnesium sulfate. After distilling off the solvent under a decreased pressure, the residue was purified by a column chromatography (eluate: a mixture of toluene and heptane = 1:9) and recrystallization for three times from 20m-1 of ethanol), to obtain the title compound as a white solid (0.011 mol). Various spectra data thereof support the structure well.

### Example 2

Preparation of 4-4'-bis(6,6-difluoro-1,5-hexadienyl)bicyclohexane 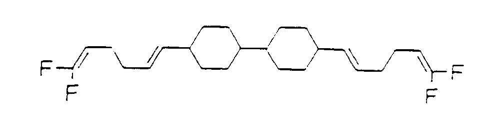 (Compound No. 2, a compound according to the formula (1) wherein R₁ and R₂ are 6,6-difluoro-1,5-hexadienyl groups, X₃ is a covalent bond, C and D are 1,4-cyclohexane rings, and m=n=0).

A mixture of methoxy methyl triphenyl phosphonium chloride (0.1 mol), potassium-t-butoxide (0.11 mol) and 300ml of THF was stirred for 2 hours, to obtain a red solution. To it, bicycloexane-1,4-dione (0.05 mol) in 300ml of THF was added and stirred for 2 hours. To the reactant, 500ml of toluene and 500ml of water were added to carry out extraction, then an organic layer was extracted twice with 500ml of water and the solvent was distilled off under a decreased pressure from the organic layer. 1L of heptane was added to the residue and stirred well to filter off the undissolved materials. Heptane was distilled off under a decreased presure, to obtain the residue as a white solid.

300ml of THF and 150ml of 6N-HCl were added and stirred for 12 hours. The reactant was extracted with 300ml of toluene, washed for three times with 500ml of water, and thereafter dried on anhydrous magnesium sulfate. The solvent was distilled off under a decreased pressure, to obtain bicyclohexane-4,4'-dicarboaldehyde (0.03 mol) as a white solid. The said material was used for the subsequent reaction after dried sufficiently.

Potassium-t-butoxide (0.02 mol) was added to a mixture of 2-1,3-difluoro-2-yl) ethyl triphenyl phosphonium bromide (0.02 mol) and 200ml of THF and stirred for 1 hour, to which 100ml of a solution of bicyclohexane-4,4'-dicarboaldehyde (0.01 mol) in THF was added dropwise for 2 hours at below -20 °C and stirred for 2 hours. 100ml of toluene and 100ml of water were added to the reactant to carry out extraction, then an organic layer was extracted for three times with 100ml of water, and the solvent was distillled off under a decreased pressure from the organic layer.

500ml of heptane was added to the residue and stirred, and the insolubles were filtered off. To it, 10ml of formic acid and 100ml of toluene were added and refluxed for 2 hours. The reactant was extracted with 200ml of toluene, washed with water for three times, and thereafter dried with anhydrous magnesium sulfate. The solvent was distilled off under a decreased pressure, to obtain 4,4'-bis(5-oxo-1-pentenyl)bicyclohexane (0.007 mol) as a white solid.

A mixture of 4,4'-bis(5-oxo-1-pentenyl)bicyclohexane (0.007 mol), sodium chlorodifluoroacetate (0.01 mol) and 80ml of DMF was stirred for 2 hours within a range of 100 ∼ 120 °C, to obtain a black solution. 200ml of heptane was added to carry out extraction, and an organic layer was washed twice with 100ml of water. The solvent was distilled off under a decreased pressure, and the residue was purified by a column chromatography (eluate: heptane) and recrystallization (benzene:ethanol = 1:1, twice), to obtain the title compound (0.005 mol). Various spectra data thereof support the structure well.

### Example 3

The following compounds according to the formula (1) are prepared similar to Examples 1 and 2.

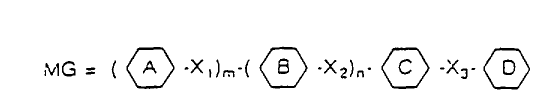

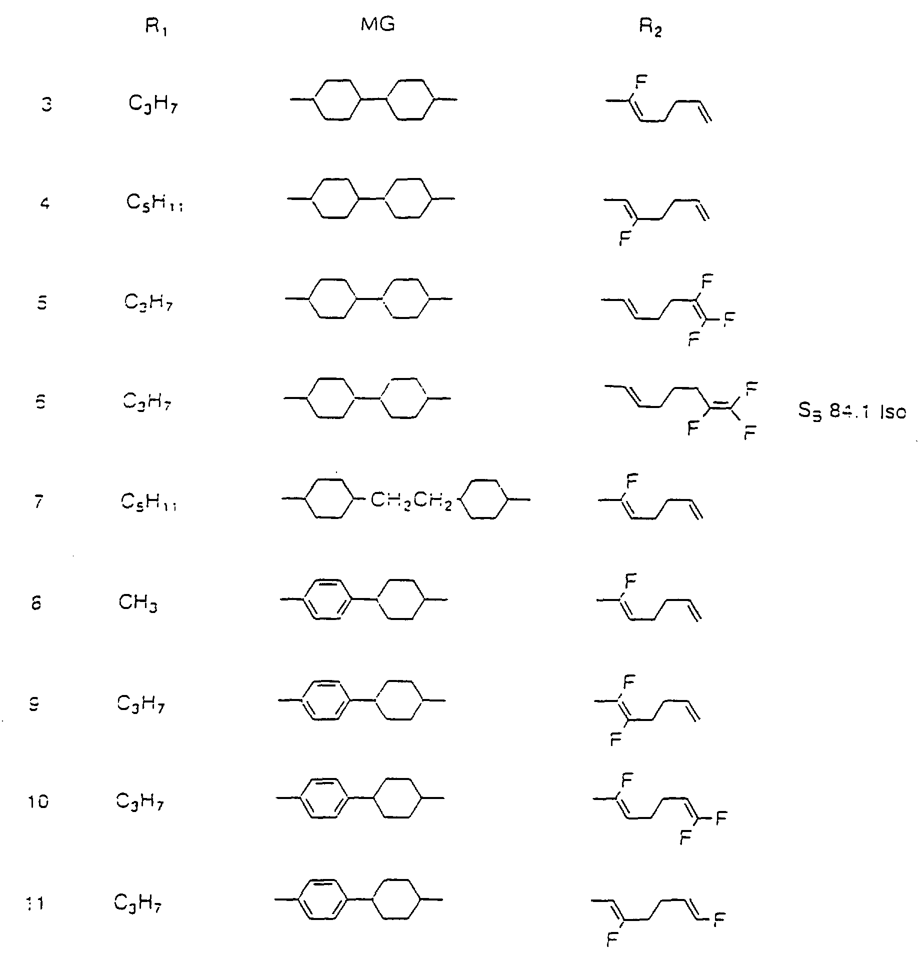

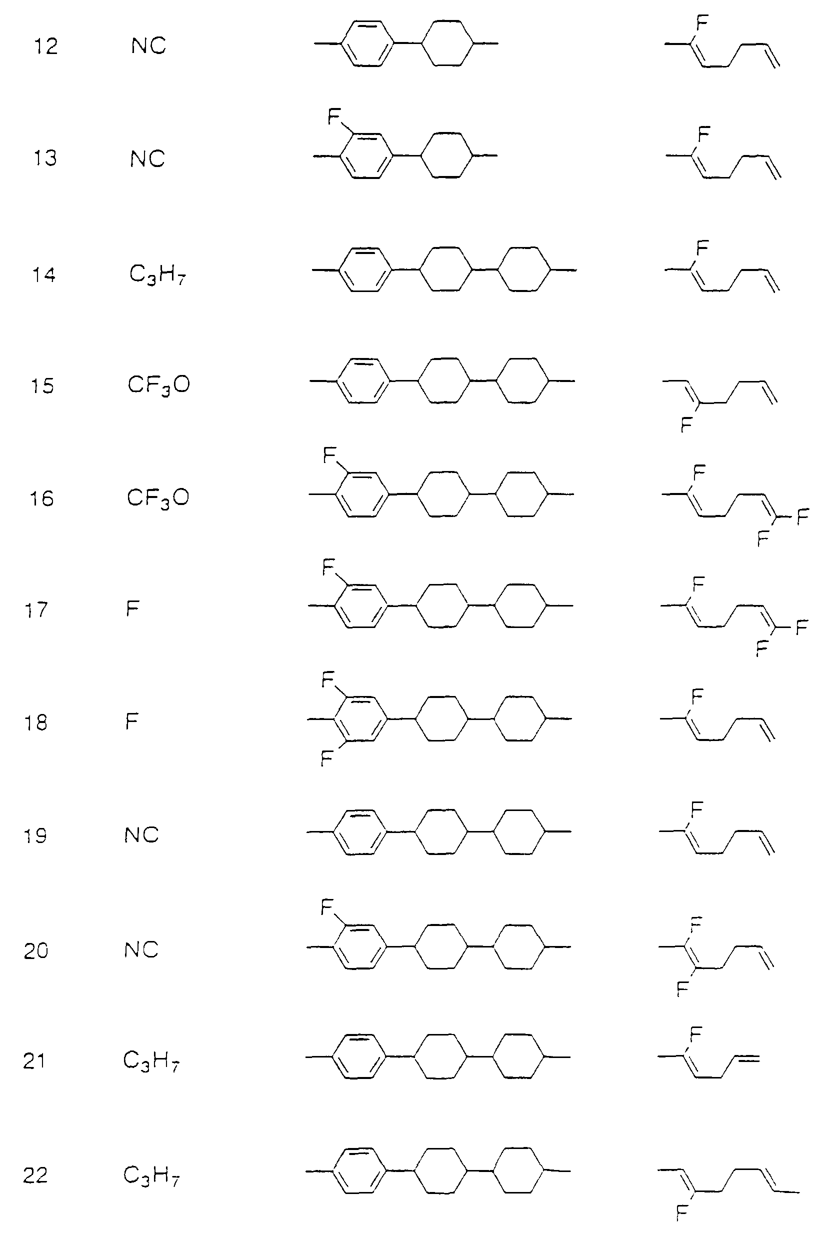

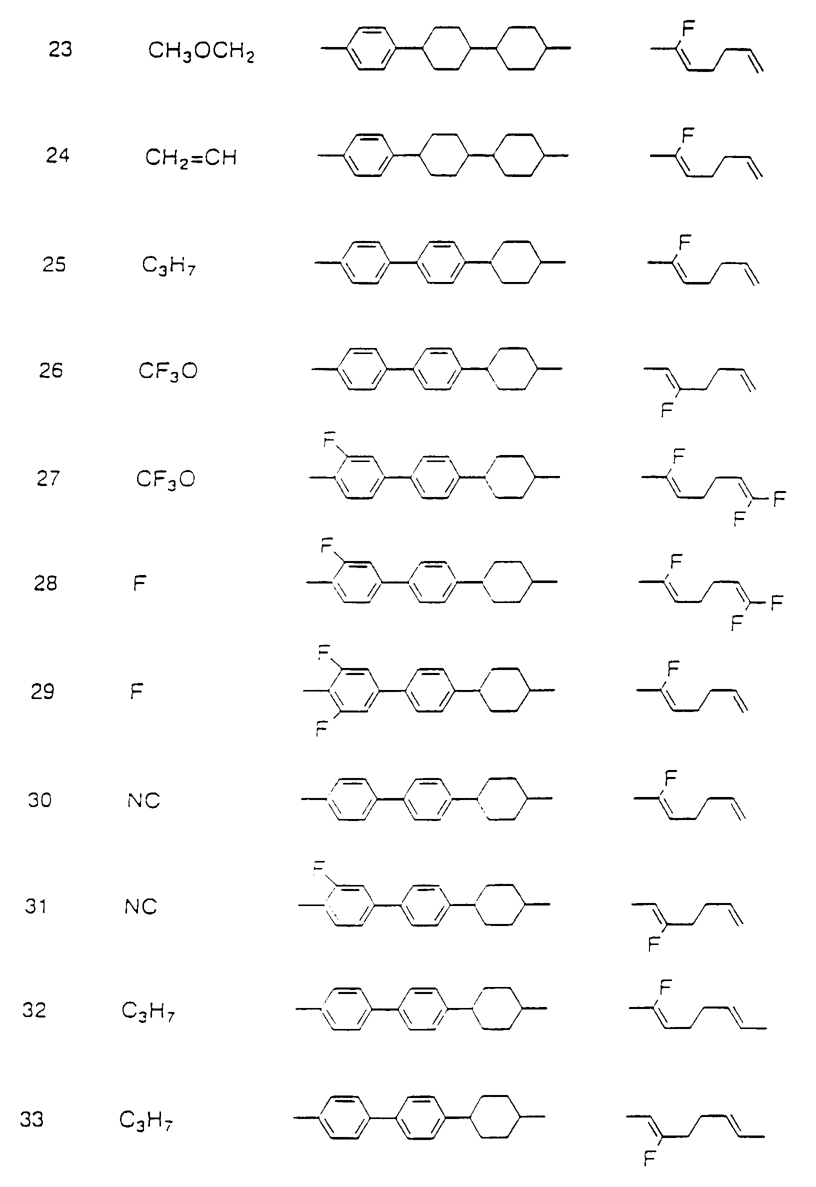

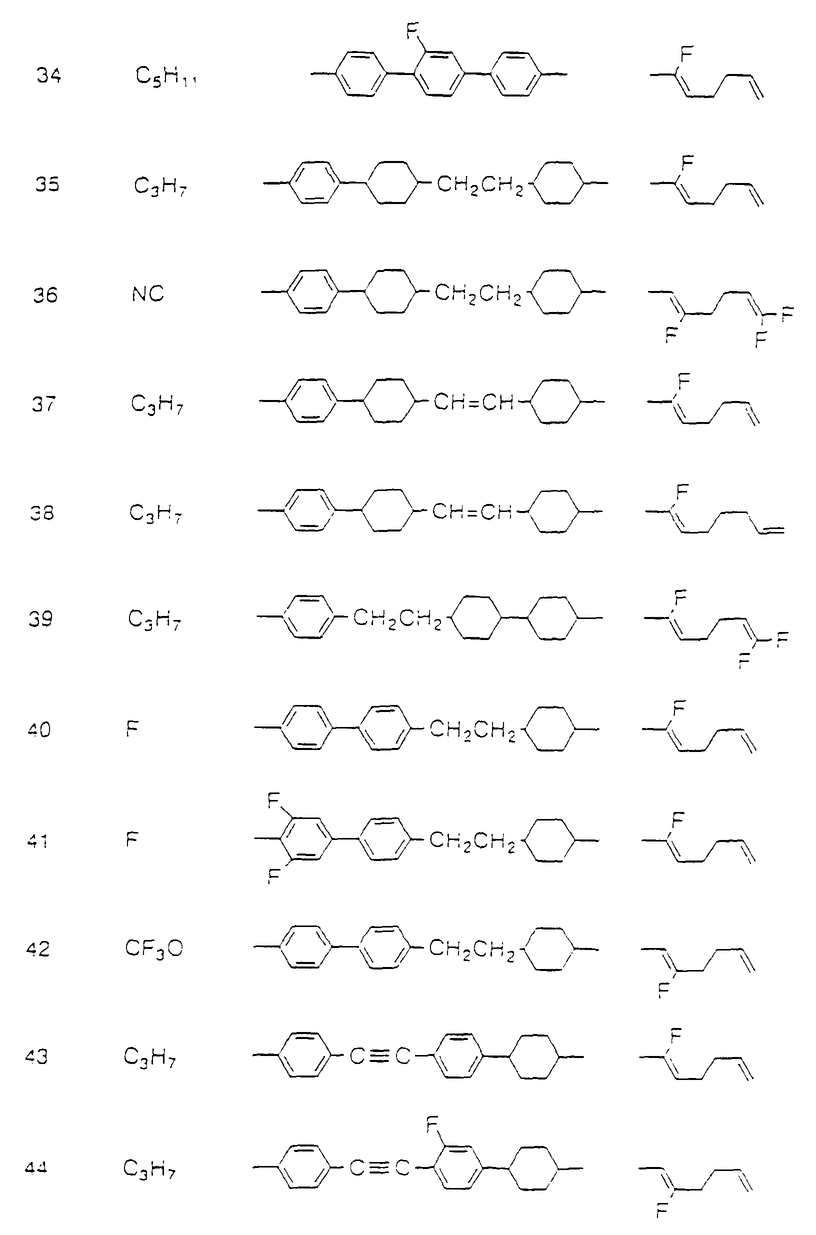

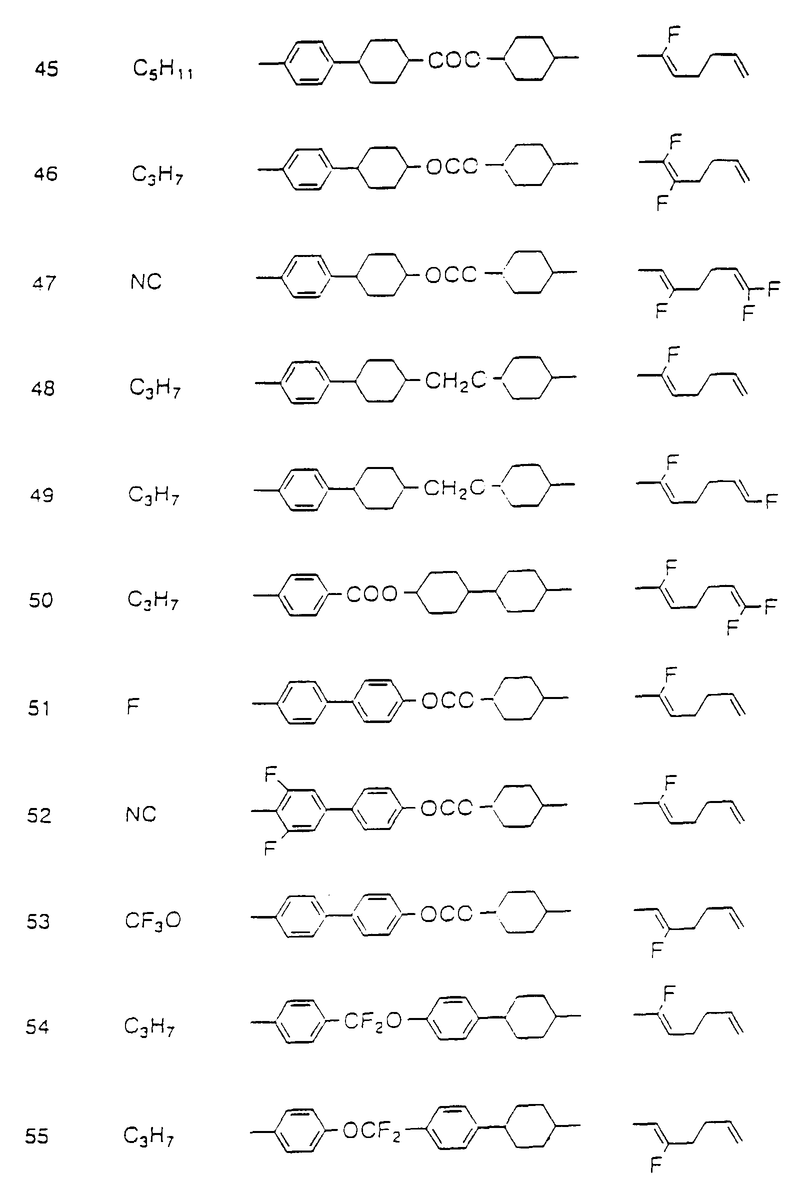

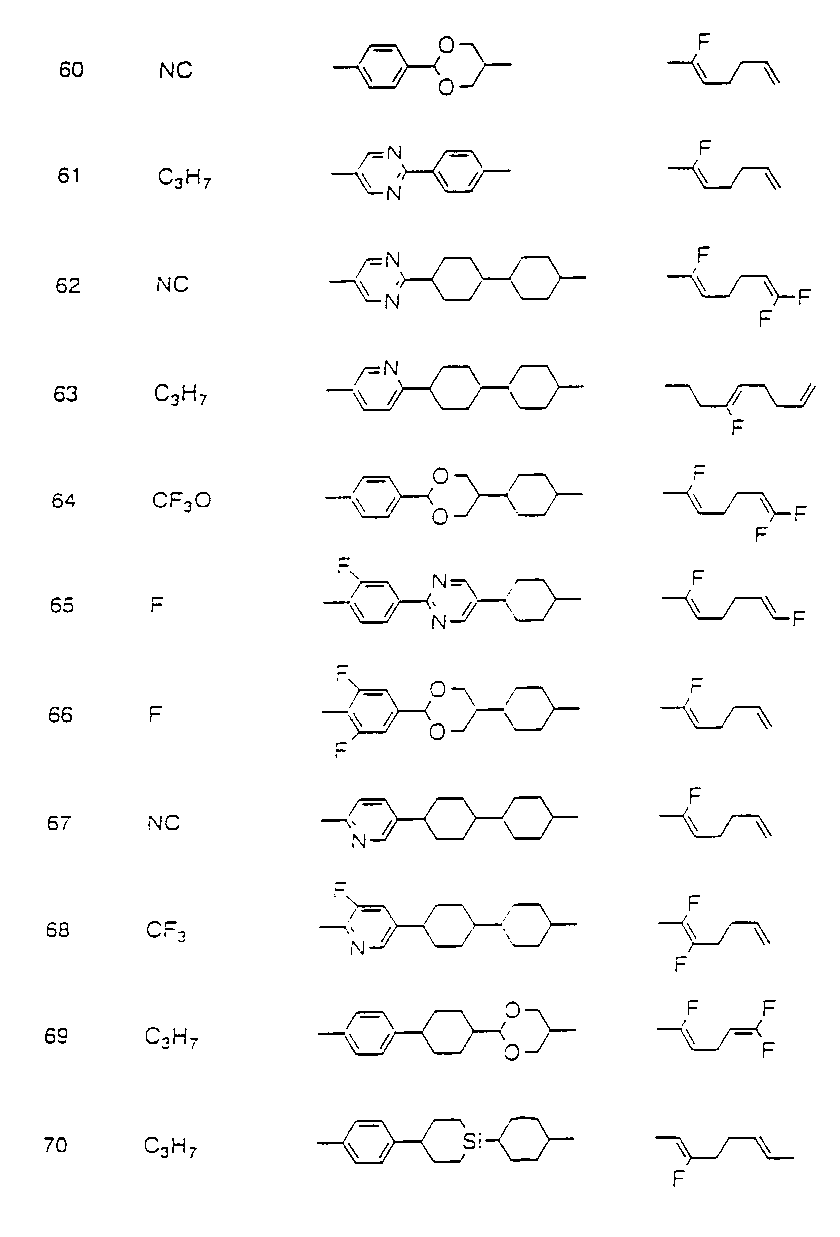

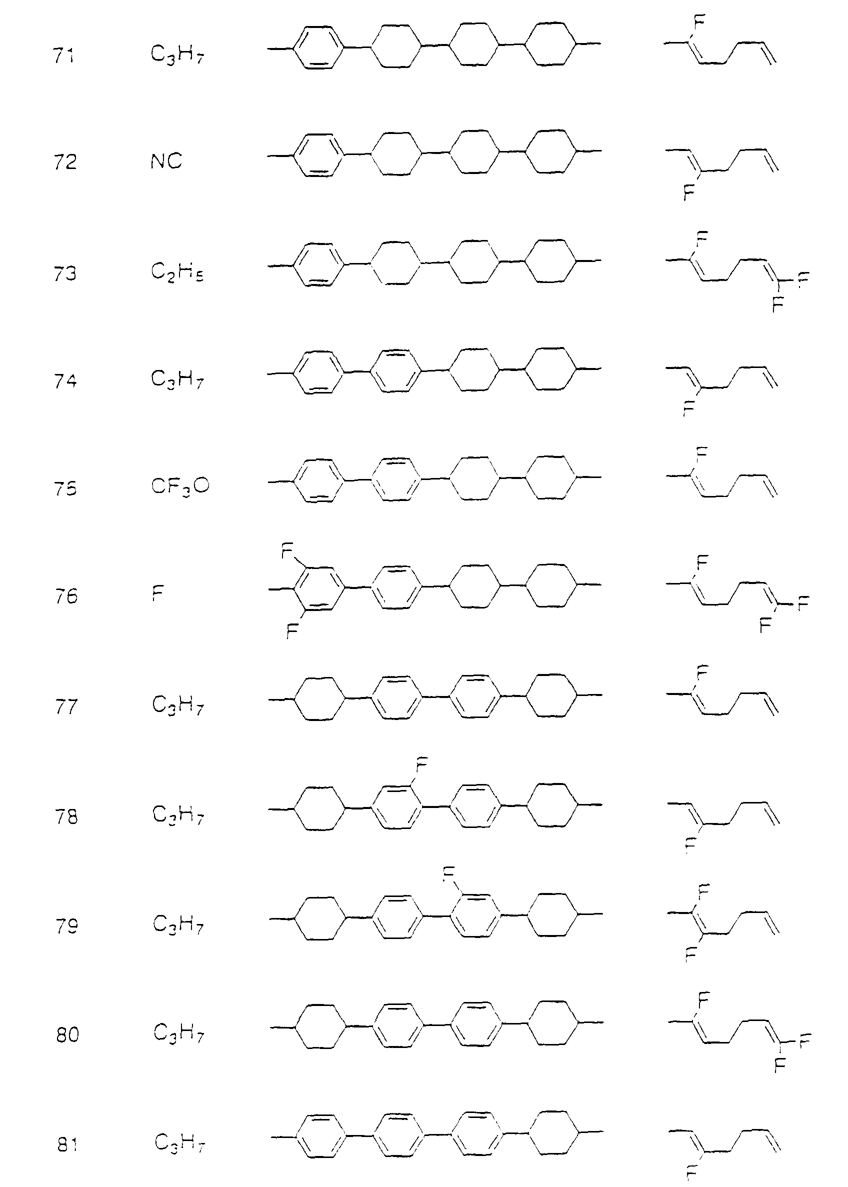

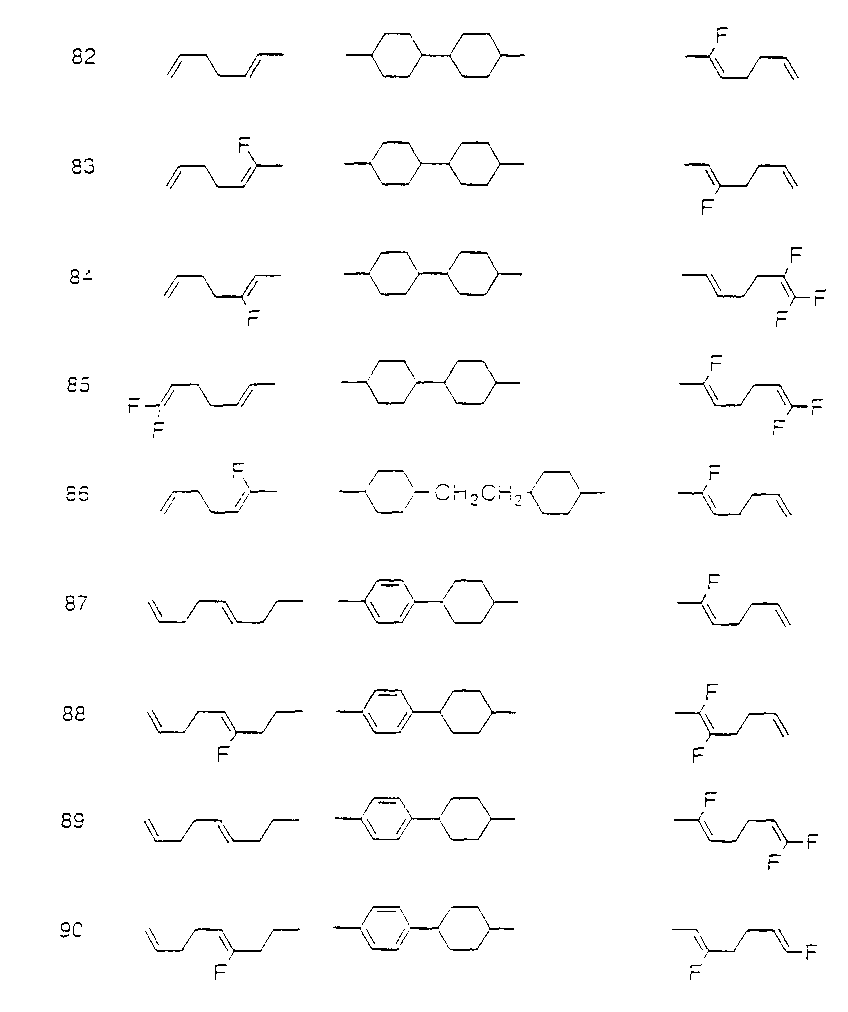

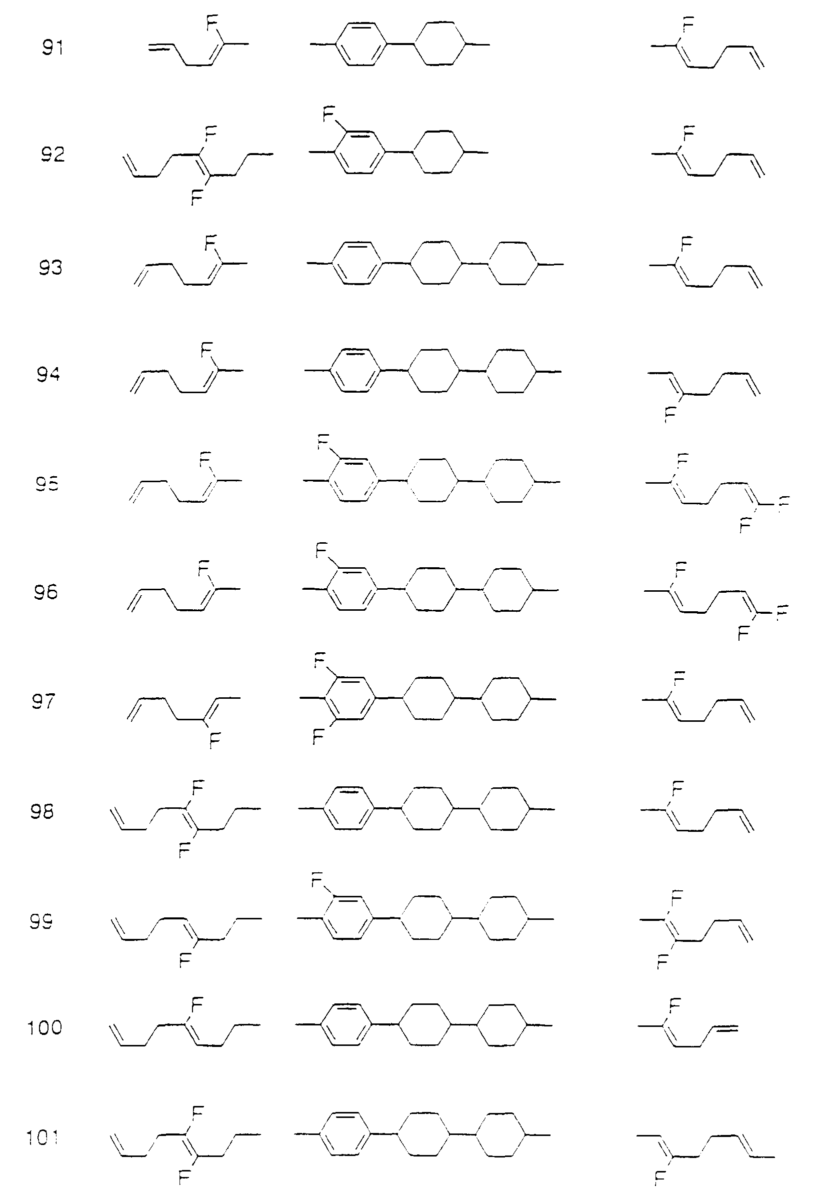

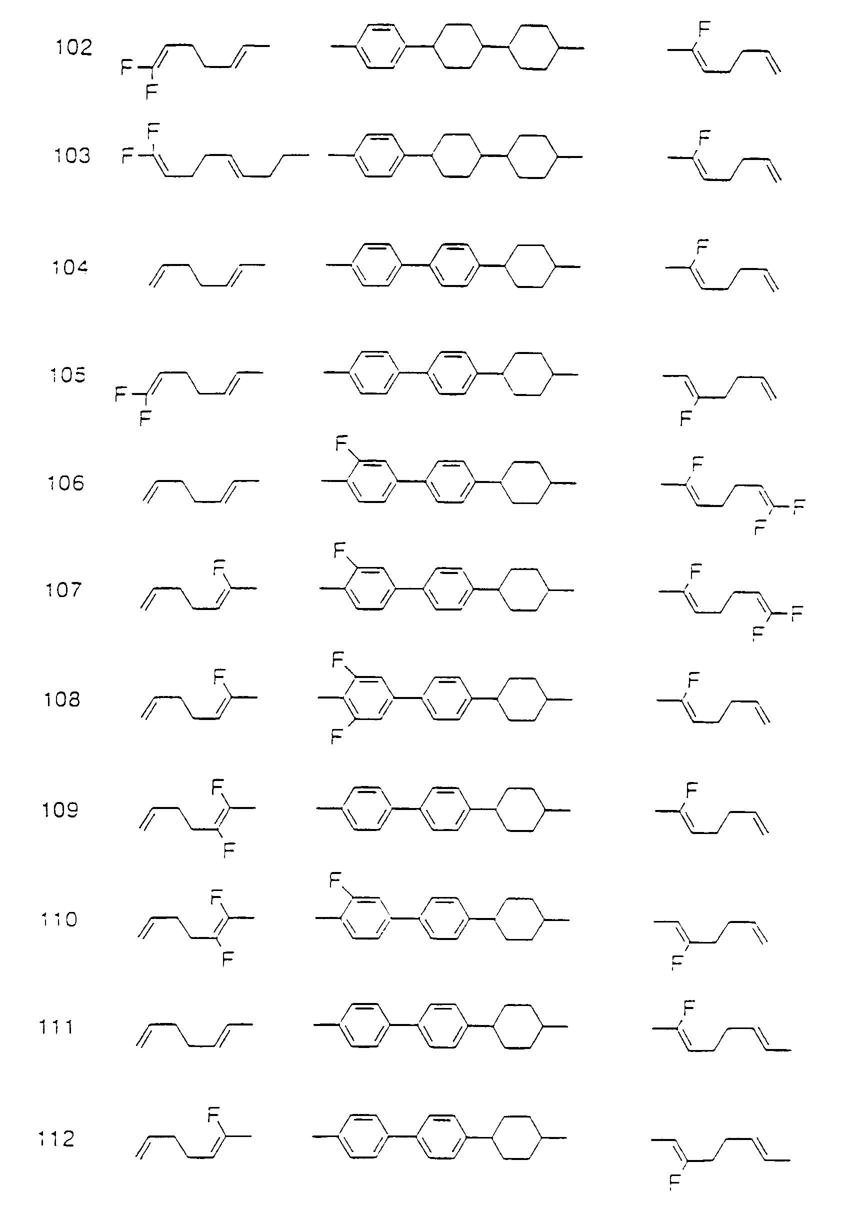

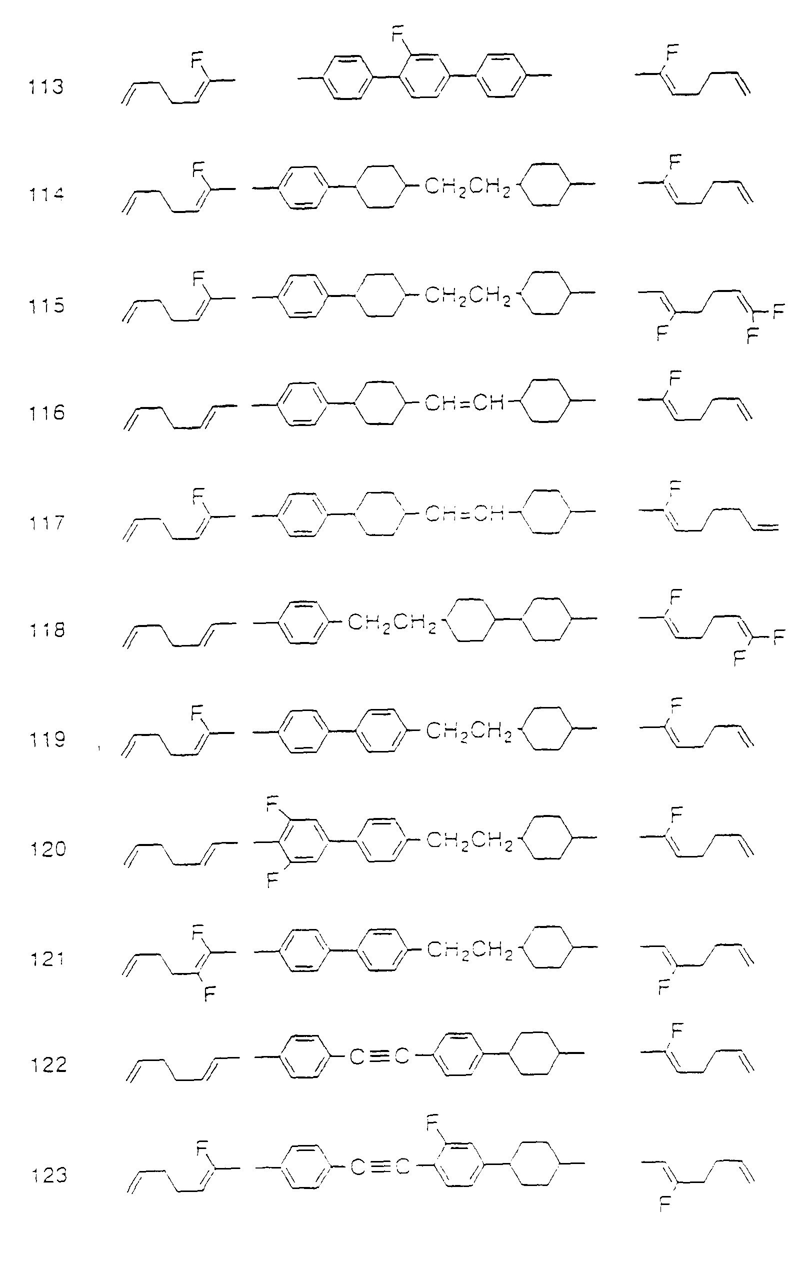

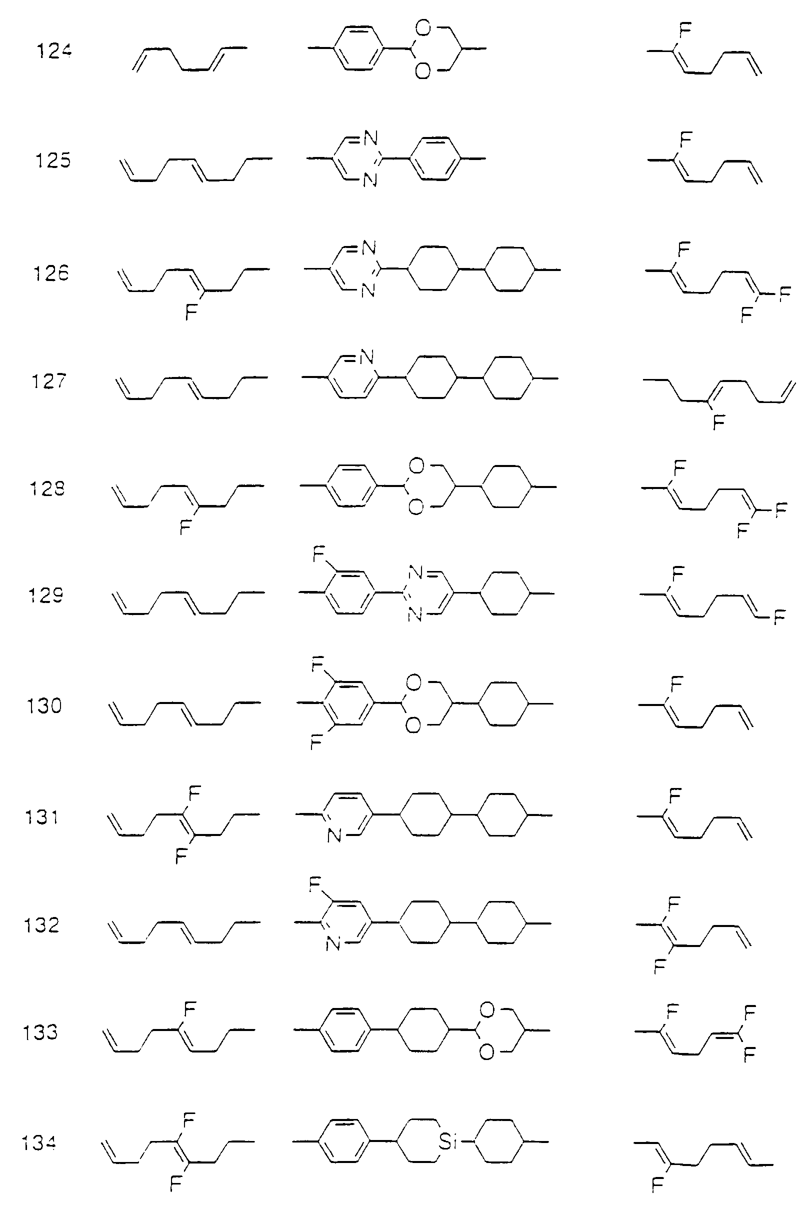

### Effect of the Invention

A compound according to the present invention shows very high elastic constant ratio K33/K11 and also shows extremely low viscosity. It has approximately same degree of high elastic constant ratio K33/K11 and lower viscosity compared to those of alkenyl compounds (17) described in Toku-Kai-Sho 61-83136 and Toku-Kai-Sho 61-56137 etc., which are used now mostly and have high elastic constant ratio K33/K11 and low viscosity.

Furthermore, the compound according to the present invention shows high stability and has sufficient chemical stability for use in a practical liquid crystalline composition.

By using the compound (1) according to the present invention with such superior characteristics, a liquid crystalline composition also with superior physical properties, namely sharp threshold value characteristics and rapid response speed, can be obtained.

## Claims

1. A liquid crystalline compound expressed by the following general formula (1) 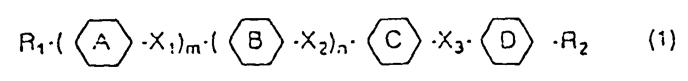
W = - (CH₂)ₚ - CX₄ = CX₅ - (CH₂)_{q} - CX₆ = C - X₇X₈ (1-1),
(wherein, R₁ and R₂ are each independently a cyano group, a halogen atom, an alkyl group with from 1 to 20 carbon atoms, a halogenated alkyl group with from 1 to 20 carbon atoms or a dienyl group shown by the general formula (1-1), in which one or non-adjacent two CH₂ group(s) may be replaced with (an) oxygen atom(s), -CH=CH- group(s) or -C≡C- group(s) and furthermore(a) hydrogen atom(s) may be replaced with (a) fluorine atom(s), X₁, X₂ and X₃ are independently each other -CH₂CH₂-, -CO-O-, -O-CO-, -CH=CH-, -C≡C-, -(CH₂)₄-, -CF₂O-, -OCF₂-, -CH₂O-, -OCH₂- or a covalent bond, rings A, B, C and D denote each independently a 1,4 phenylene ring or a 1,4-cyclohexylene ring. a bicyclo[1.1.0]butane ring, a bicyclo[1.1.1]pentane ring, a bicyclo-[2.2.2]octane ring, a cyclobutane ring, a spiro[3.3]heptane ring, in which (a)hydrogen atom(s) in the said rings may be substituted, and (a) carbon atom(s) in the said rings may be replaced with (a) nitrogen atom(s), (an) oxygen atom(s) or (a) silicon atom(s), X₄, X₅, X₆, X₇ and X₈ denote each independently a hydrogen atom, a halogen atom or an alkyl group with from one to five carbon atoms, in which at least one of them denotes a halogen atom, m and n are independently each other 0 or 1, and p is from 0 to 5, and q is from 1 to 5, provided that at least one of R₁ and R₂ is said dienyl group shown by the general formula (1-1)).

2. A liquid crystalline compound according to Claim 1, wherein X₄ is a fluorine atom.

3. A liquid crystalline compound according to Claim 1, wherein X₅ is a fluorine atom.

4. A liquid crystalline compound according to Claim 1, wherein X₆ is a fluorine atom.

5. A liquid crystalline composition consisting of at least two components, characterized in that a liquid crystalline compound expressed by the general formula (1) as described in any one of claims 1 to 4 is contained as at least one component.

6. A liquid crystalline composition, characterized in that at least one compound according to Claims 1 to 4 is contained as the first component, and one or more than one compound(s) selected from a group consisting of the general formula (2), (3) and (4): 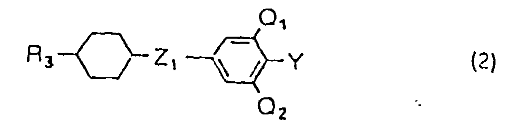 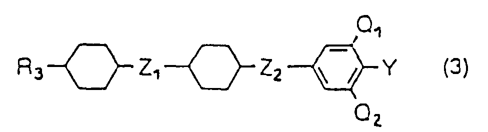 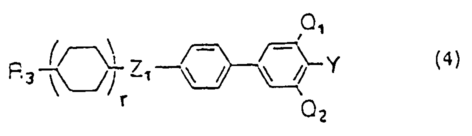 (wherein, R₃ denotes an alkyl group with from 1 to 10 carbon atoms, Y denotes a fluorine atom or a chlorine atom, Q₁ and Q₂ denote each independently a hydrogen atom or a fluorine atom, r denotes 1 or 2, and Z₁ and Z₂ denote each independently -CH₂CH₂- or a covalent bond)
is (are) contained as the second component.

7. A liquid crystalline composition, characterized in that at least one compound according to Claims 1 to 4 is contained as the first component, and one or more than one compound(s) selected from a group consisting of the general formulae (5), (6), (7), (8) and (9): 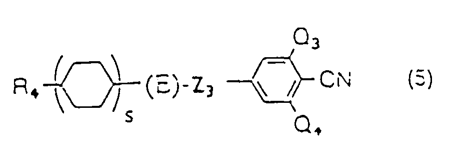 (wherein, R₄ denotes an alkyl group with from 1 to 10 carbon atoms or an alkenyl group with from 2 to 10 carbon atoms, in which (an) optional methylene group(s) of them may be substituted by (an) oxygen atom(s) in each cases, but two or more than two methylene atoms may not be substituted continuously, Z₃ denotes -CH₂CH₂-, -COO- or a covalent bonds Q₃ and Q₄ denote a hydrogen atom or a fluorine atom, E denotes a cyclohexane ring, a benzene ring or a 1,3-dioxan ring, and s denotes 0 or 1), 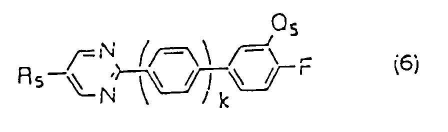 (wherein, R₅ denotes an alkyl group with from 1 to 10 carbon atoms, Q₅ denotes a hydrogen atom or a fluorine atom, and k denotes 0 or 1), 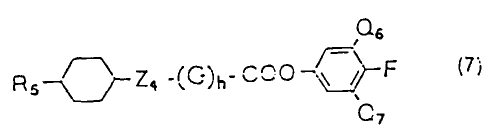 (wherein, R₆ denotes an alkyl group with from 1 to 10 carbon atoms, G denotes a cyclohexane ring or a benzene ring, Q₆ and Q₇ denote each independently a hydrogen atom or a fluorine atom, Z₄ denotes -COO- or a covalent bond, and h denotes 0 or 1),
R₇ - (H) - Z₅ - (J) - R₈ (8)
(wherein, R₇ and R₈ denote each independently an alkyl group, an alkoxy group or an alkoxymethyl group with from 1 to 10 carbon atoms, H denotes a cyclohexane ring, a pyrimidine ring or a benzene ring, J denotes a cyclohexane ring or a benzene ring, Z₆ denotes -C≡C-, -COO-, -CH₂CH₂- or a covalent bond), 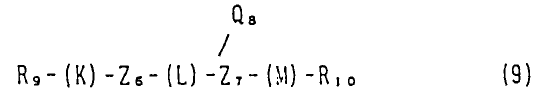 (wherein, R₉ denotes an alkyl group or an alkoxy group with from 1 to 10 carbon atoms, R₁₀ denotes an alkyl group, an alkoxyl group or an alkoxymethyl group with from 1 to 10 carbon atoms, K denotes a cyclohexane ring or a pyrimidine ring, L and M denote each independently a cyclohexane ring or a benzene ring, Z₆ denotes -COO-, -CH₂CH₂- or a covalent bond, Z₇ denotes -C≡C-, -COO- or a covalent bond, and Q₈ denotes a hydrogen atom or a fluorine atom)
is (are) contained as the second component.

8. A liquid crystalline display element with use of a liquid crystalline composition consisting of at least two components, characterized in that a liquid crystalline compound expressed by the general formula (1) is contained as at least one component.

9. A liquid crystalline display element with use of a liquid crystalline composition according to Claims 5 to 8.

## Patentansprüche

1. Flüssigkristalline Verbindung der folgenden allgemeinen Formel (1): 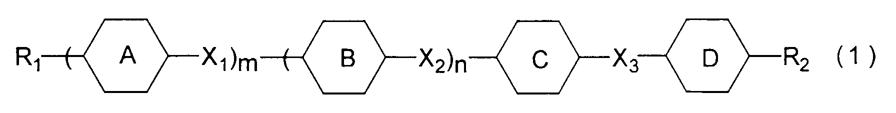
W = -(CH₂)ₚ-CX₄=CX₅-(CH₂)_{q}-CX₆=C-X₇X₈ (1-1)
worin R₁ und R₂ jeweils unabhängig voneinander eine Cyanogruppe, ein Halogenatom, eine Alkylgruppe mit 1 bis 20 Kohlenstoffatomen, eine halogenierte Alkylgruppe mit 1 bis 20 Kohlenstoffatomen oder eine Dienylgruppe der allgemeinen Formel (1-1), worin eine oder zwei nicht-benachbarte CH₂-Gruppe(n) durch (ein) Sauerstoffatom(e), (eine) -CH=CH-Gruppe(n) oder -C≡C-Gruppe(n) ersetzt sein kann/können, und ferner (ein) Wasserstoffatom(e) durch (ein) Fluoratom(e) ersetzt sein kann/können, repräsentieren, X₁, X₂ und X₃ sind jeweils unabhängig voneinander -CH₂CH₂-, -CO-O-, -O-CO-, -CH=CH-, -C≡C-, -(CH₂)₄-, -CF₂O-, -OCF₂-, -CH₂O-, -OCH₂-oder eine kovalente Bindung, die Ringe A, B, C und D kennzeichnen jeweils unabhängig voneinander einen 1,4-Phenylenring oder einen 1,4-Cyclohexylenring, einen Bicyclo[1.1.0]butan-Ring, einen Bicyclo[1.1.1]pentan-Ring, einen Bicyclo[2.2.2]octan-Ring, einen Cyclobutanring, einen Spiro [3.3] heptan-Ring, worin (ein) Wasserstoffatom(e) in diesen Ringen substituiert sein kann/können, und (ein) Kohlenstoffatom(e) in den Ringen ersetzt sein kann/können durch (ein) Stickstoffatom(e), (ein) Sauerstoffatom(e) oder (ein) Siliciumatom(e), X₄, X₅, X₆, X₇ und X₈ kennzeichnen jeweils unabhängig voneinander ein Wasserstoffatom, ein Halogenatom oder eine Alkylgruppe mit 1 bis 5 Kohlenstoffatomen, worin mindestens eines unter diesen ein Halogenatom darstellt, m und n sind unabhängig voneinander jeweils 0 oder 1, p ist 0 bis 5 und q ist 1 bis 5, mit der Massgabe, dass mindestens eines von R₁ und R₂ die Dienylgruppe der allgemeinen Formel (1-1) ist.

2. Flüssigkristalline Verbindung gemäss Anspruch 1, worin X₄ ein Fluoratom ist.

3. Flüssigkristalline Verbindung gemäss Anspruch 1, worin X₅ ein Fluoratom ist.

4. Flüssigkristalline Verbindung gemäss Anspruch 1, worin X₆ ein Fluoratom ist.

5. Flüssigkristallzusammensetzung, bestehend aus mindestens zwei Komponenten, dadurch gekennzeichnet, dass eine flüssigkristalline Verbindung der allgemeinen Formel (1), wie in mindestens einem der Ansprüche 1 bis 4 beschrieben, als mindestens eine Komponente enthalten ist.

6. Flüssigkristallzusammensetzung, dadurch gekennzeichnet, dass mindestens eine Verbindung gemäss den Ansprüchen 1 bis 4 als erste Komponente enthalten ist, und eine oder mehrere Verbindung(en), ausgewählt aus den allgemeinen Formeln (2), (3) und (4), als zweite Komponente enthalten ist (sind): 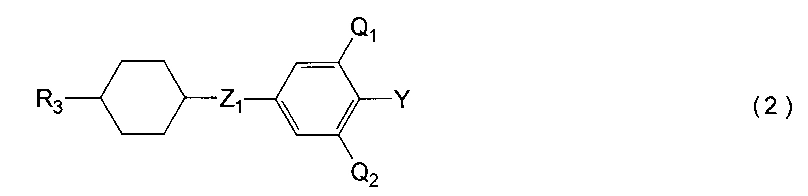 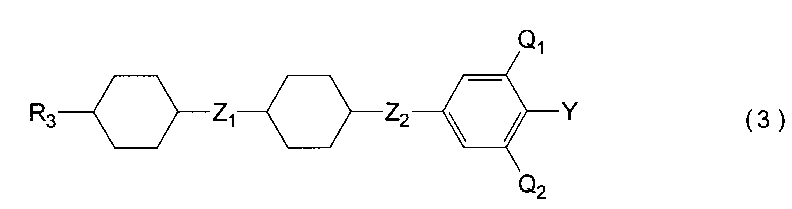 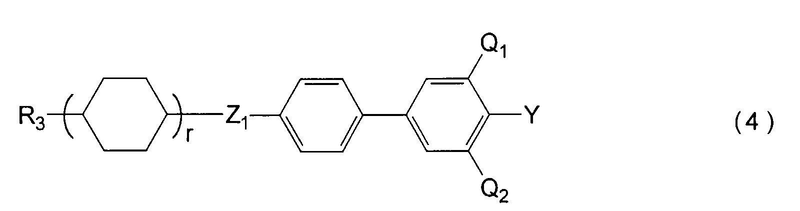 worin R₃ eine Alkylgruppe mit 1 bis 10 Kohlenstoffatomen ist, Y ist ein Fluoratom oder ein Chloratom, Q₁ und Q₂ sind jeweils unabhängig voneinander ein Wasserstoffatom oder ein Fluoratom, r ist 1 oder 2 und Z₁ und Z₂ sind unabhängig voneinander -CH₂CH₂- oder eine kovalente Bindung.

7. Flüssigkristallzusammensetzung, dadurch gekennzeichnet, dass mindestens eine Verbindung gemäss den Ansprüchen 1 bis 4 als erste Komponente enthalten ist, und eine oder mehrere Verbindung(en), ausgewählt aus den allgemeinen Formeln (5), (6), (7), (8) und (9), als zweite Komponente enthalten ist (sind): 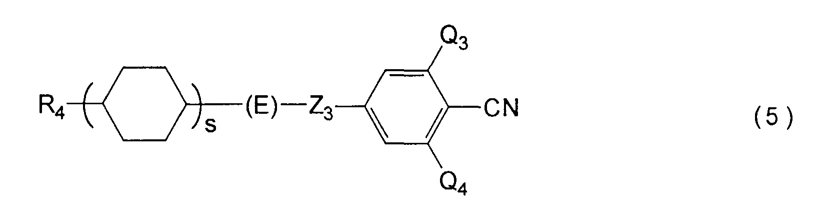 worin R₄ eine Alkylgruppe mit 1 bis 10 Kohlenstoffatomen oder eine Alkenylgruppe mit 2 bis 10 Kohlenstoffatomen ist, worin wahlweise (eine) Methylengruppe(n) unter diesen ersetzt sein kann durch (ein) Sauerstoffatom(e), aber nicht zwei oder mehr als zwei Methylengruppen in Folge ausgetauscht sein dürfen, Z₃ ist -CH₂CH₂-, -COO- oder eine kovalente Bindung, Q₃ und Q₄ sind ein Wasserstoffatom oder ein Fluoratom, E ist ein Cyclohexanring, ein Benzolring oder ein 1,3-Dioxan-Ring und s ist 0 oder 1, 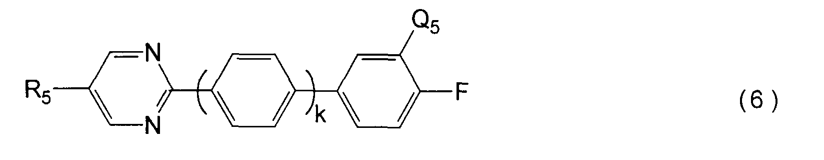 worin R₅ eine Alkylgruppe mit 1 bis 10 Kohlenstoffatomen ist, Q₅ ist ein Wasserstoffatom oder ein Fluoratom und k ist 0 oder 1, 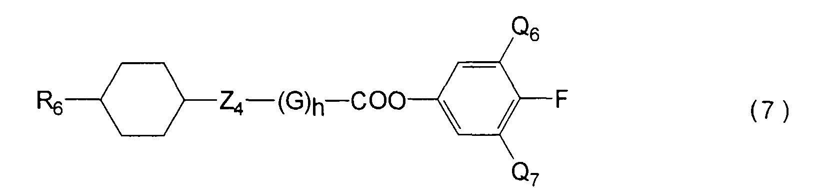 worin R₆ eine Alkylgruppe mit 1 bis 10 Kohlenstoffatomen ist, G ist ein Cyclohexanring oder ein Benzolring, Q₆ und Q₇ sind jeweils unabhängig voneinander ein Wasserstoffatom oder ein Fluoratom, Z₄ ist -COO- oder eine kovalente Bindung, und h ist 0 oder 1,
R₇-(H)-Z₅-(J)-R₈ (8)
worin R₇ und R₈ jeweils unabhängig voneinander eine Alkoxygruppe oder eine Alkoxymethylgruppe mit 1 bis 10 Kohlenstoffatomen sind, H ist ein Cyclohexanring, ein Pyrimidinring oder ein Benzolring, J ist ein Cyclohexanring oder ein Benzolring, Z₅ ist -C≡C-, -COO-, -CH₂CH₂- oder eine kovalente Bindung, 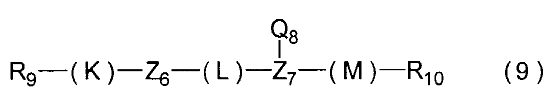 worin R₉ eine Alkylgruppe oder eine Alkoxygruppe mit 1 bis 10 Kohlenstoffatomen ist, R₁₀ ist eine Alkylgruppe, eine Alkoxylgruppe oder eine Alkoxymethylgruppe mit 1 bis 10 Kohlenstoffatomen, K ist ein Cyclohexanring oder ein Pyrimidinring, L und M sind jeweils unabhängig voneinander ein Cyclohexanring oder ein Benzolring, Z₆ ist -COO-, -CH₂CH₂-, oder eine kovalente Bindung, Z₇ ist -C≡C-, -COO- oder eine kovalente Bindung, und Q₈ ist ein Wasserstoffatom oder ein Fluoratom.

8. Flüssigkristall-Anzeigeelement unter Verwendung einer Flüssigkristallzusammensetzung, die aus mindestens zwei Komponenten besteht, dadurch gekennzeichnet, dass eine flüssigkristalline Verbindung der allgemeinen Formel (1) als mindestens eine Komponente enthalten ist.

9. Flüssigkristall-Anzeigeelement unter Verwendung einer Flüssigkristallzusammensetzung gemäss den Ansprüchen 5 bis 8.

## Revendications

1. Composé cristallin liquide exprimé par la formule générale suivante (1) 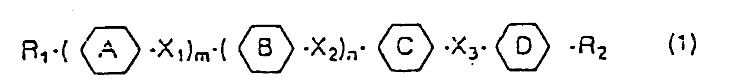
W = -(CH₂)ₚ-CX₄ = CX₅ - (CH₂)_{q} - CX₆ = C - X₇X₈ (1-1),
(dans laquelle R₁ et R₂ sont chacun indépendamment un groupement cyano, un atome d'halogène, un groupement alkyle comportant de 1 à 20 atomes de carbone, un groupement alkyle halogéné comportant de 1 à 20 atomes de carbone ou un groupement diényle représenté par la formule générale (1-1), dans laquelle un groupement CH₂ ou deux groupements CH₂ non adjacents peuvent être remplacés par un ou des atomes d'oxygène, un ou des groupements -CH=CH-, ou bien un ou des groupements -C≡C- et en outre un ou des atomes d'hydrogène peuvent être remplacés par un ou des atomes de fluor, X₁, X₂ et X₃ sont indépendamment l'un de l'autre -CH₂CH₂-, -CO-O-, -O-CO-, -CH=CH-, -C≡C-, -(CH₂)₄-, -CF₂O-, -OCF₂-, -CH₂O-, -OCH₂- ou bien une liaison covalente, les cycles A, B, C et D représentent chacun indépendamment un cycle 1,4-phénylène ou un cycle 1,4-cyclohexylène, un cycle bicyclo[1.1.0]butane, un cycle bicyclo[1.1.1]pentane, un cycle bicyclo[2.2.2]octane, un cycle cyclobutane, un cycle spiro[3.3]heptane, dans lesquels un ou des atomes d'hydrogène dans lesdits cycles peuvent être substitués, et un ou des atomes de carbone dans lesdits cycles peuvent être remplacés par un ou des atomes d'azote, un ou des atomes d'oxygène ou bien un ou des atomes de silicium, X₄, X₅, X₆, X₇ et X₈ représentent chacun indépendamment un atome d'hydrogène, un atome d'halogène ou un groupement alkyle comportant de un à cinq atomes de carbone, dans lesquels au moins l'un d'eux représente un atome d'halogène, m et n prennent chacun indépendamment l'un de l'autre la valeur 0 ou 1, et p prend une valeur de 0 à 5, et q prend une valeur de 1 à 5, à la condition qu'au moins l'un de R₁ et R₁ soit ledit groupement diényle représenté par la formule générale (1-1)).

2. Composé cristallin liquide selon la revendication 1, dans lequel X₄ est un atome de fluor.

3. Composé cristallin liquide selon la revendication 1, dans lequel X₅ est un atome de fluor.

4. Composé cristallin liquide selon la revendication 1, dans lequel X₆ est un atome de fluor.

5. Composition cristalline liquide constituée d'au moins deux constituants, caractérisée en ce qu'un composé cristallin liquide exprimé par la formule générale (1) tel que décrit dans l'une quelconque des revendications 1 à 4 est contenu en tant qu'au moins un constituant.

6. Composition cristalline liquide, caractérisée en ce qu'au -moins un composé conforme aux revendications 1 à 4 est contenu en tant que premier constituant, et un ou plus d'un composé choisis parmi un groupe constitué des formules générales (2), (3) et (4) : 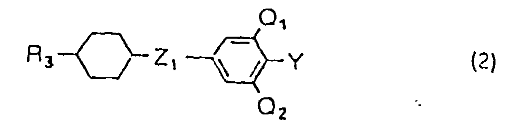 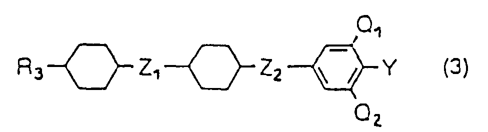 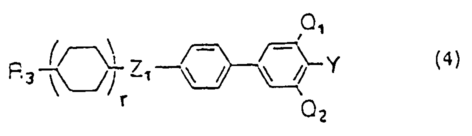 (dans lesquelles R₃ représente un groupement alkyle comportant de 1 à 10 atomes de carbone, Y représente un atome de fluor ou un atome de chlore, Q₁ et Q₂ représentent chacun indépendamment un atome d'hydrogène ou un atome de fluor, r prend la valeur 1 ou 2, et Z₁ et Z₂ représentent chacun indépendamment -CH₂CH₂- ou bien une liaison covalente)
sont contenus en tant que second constituant.

7. Composition cristalline liquide, caractérisée en ce qu'au moins un composé conforme aux revendications 1 à 4 est contenu en tant que premier constituant, et un ou plus d'un composé choisis parmi un groupe constitué des formules générales (5), (6), (7), (8) et (9) : 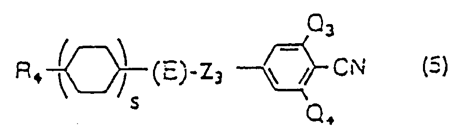 (dans laquelle R₄ représente un groupement alkyle comportant de 1 à 10 atomes de carbone ou un groupement alcényle comportant de 2 à 10 atomes de carbone, dans lesquels un ou des groupements méthylène optionnels de ceux-ci peuvent être substitués par un ou des atomes d'oxygène dans chaque cas, mais deux ou plus de deux groupements méthylène ne peuvent pas être substitués en succession, Z₃ représente -CH₂CH₂-, -COO- ou bien une liaison covalente, Q₃ et Q₄ représentent un atome d'hydrogène ou un atome de fluor, E représente un cycle cyclohexane, un cycle benzénique ou bien un cycle 1,3-dioxane et s prend la valeur 0 ou 1), 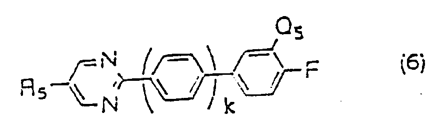 (dans laquelle R₅ représente un groupement alkyle comportant de 1 à 10 atomes de carbone, Q₅ représente un atome d'hydrogène ou un atome de fluor, et k prend la valeur 0 ou 1), 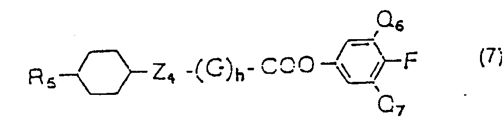 (dans laquelle R₅ représente un groupement alkyle comportant de 1 à 10 atomes de carbone, G représente un cycle cyclohexane ou un cycle benzénique, Q₆ et Q₇ représentent chacun indépendamment un atome d'hydrogène ou un atome de fluor, Z₄ représente -COO- ou bien une liaison covalente, et h prend la valeur 0 ou 1),
R₇ - (H) - Z₅ - (J) - R₈ (8)
(dans laquelle R₇ et R₈ représentent chacun indépendamment un groupement alkyle, un groupement alcoxy ou un groupement alcoxyméthyle comportant de 1 à 10 atomes de carbone, H représente un cycle cyclohexane, un cycle pyrimidine ou un cycle benzénique, J représente un cycle cyclohexane ou un cycle benzénique, Z₅ représente -C≡C-, -COO-, -CH₂CH₂- ou bien une liaison covalente), 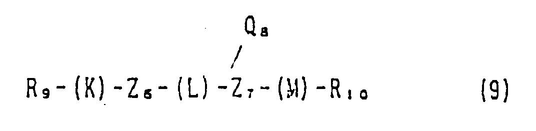 (dans laquelle R₉ représente un groupement alkyle ou un groupement alcoxy comportant de 1 à 10 atomes de carbone, R₁₀ représente un groupement alkyle, un groupement alcoxyle ou un groupement alcoxyméthyle comportant de 1 à 10 atomes de carbone, K représente un cycle cyclohexane ou un cycle pyrimidine, L et M représentent chacun indépendamment un cycle cyclohexane ou un cycle benzénique, Z₆ représente -COO-, -CH₂CH₂- ou bien une liaison covalente, Z₇ représente -C≡C-, -COO- ou bien une liaison covalente, et Q₈ représente un atome d'hydrogène ou un atome de fluor)
sont contenus en tant que second constituant.

8. Elément d'affichage à cristaux liquides utilisant une composition cristalline liquide constituée d'au moins deux constituants, caractérisé en ce qu'un composé cristallin liquide exprimé par la formule générale (1) est contenu en tant qu'au moins un constituant.

9. Elément d'affichage à cristaux liquides utilisant une composition cristalline liquide conforme aux revendications 5 à 8.
